(19) Europäisches Patentamt
European Patent Office
Office européen des brevets

(11) **EP 2 408 390 B1**

(12) **EUROPÄISCHE PATENTSCHRIFT**

(45) Veröffentlichungstag und Bekanntmachung des Hinweises auf die Patenterteilung:
**28.04.2021 Patentblatt 2021/17**

(21) Anmeldenummer: **10703876.2**

(22) Anmeldetag: **12.02.2010**

(51) Int Cl.:
*A61B 34/00* (2016.01)

(86) Internationale Anmeldenummer:
**PCT/EP2010/051771**

(87) Internationale Veröffentlichungsnummer:
**WO 2010/105882 (23.09.2010 Gazette 2010/38)**

(54) **SPULENANORDNUNG ZUR FÜHRUNG EINES MAGNETISCHEN OBJEKTS IN EINEM ARBEITSRAUM**

COIL ASSEMBLY FOR GUIDING A MAGNETIC OBJECT IN A WORKSPACE

ARRANGEMENT DE BOBINES POUR GUIDER UN OBJET MAGNÉTIQUE DANS UN ESPACE DE TRAVAIL

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **16.03.2009 DE 102009013352**

(43) Veröffentlichungstag der Anmeldung:
**25.01.2012 Patentblatt 2012/04**

(73) Patentinhaber: **Siemens Healthcare GmbH 91052 Erlangen (DE)**

(72) Erfinder: **REINSCHKE, Johannes 90425 Nürnberg (DE)**

(56) Entgegenhaltungen:
**WO-A1-2007/110278     WO-A1-2008/004497
WO-A1-2009/089992**

**Beschreibung**

Die Erfindung betrifft eine Spulenanordnung zur berührungslosen Führung eines magnetischen Objektes, insbesondere einer Endoskopiekapsel, in einem Arbeitsraum.

Die Verwendung von Endoskopen und Kathetern findet in der Medizin zur Diagnose oder zur Behandlung des Inneren eines Patienten immer breitere Anwendung. Die Instrumente werden über Körperöffnungen oder Schnitte in den Körper eingeführt und lassen sich von außen geführt in einer Längsrichtung verschieben, wofür eine mechanische Verbindung zum Instrument notwendig ist. Bei der Vorwärtsbewegung des Instruments in den Körper hinein treten jedoch an Kurven oder Verzweigungen in der Regel Schwierigkeiten bei der Navigation derart auf, dass der Bediener das Instrument ggf. durch mehrfaches Probieren in die gewollte Richtung dirigieren muss und eine Stützkraft vom Gewebe auf das Instrument für die weitere Navigation erforderlich ist. Dies ist für den Bediener mit höherem zeitlichem Aufwand und für den Patienten mit Schmerzen verbunden. Im schlechtesten Fall ist nicht auszuschließen, dass die Führung in die geplante Richtung gar nicht gelingt oder das Risiko der Gewebeperforation eintritt. Weiterhin kann es im Fall der Endoskopie von Interesse sein, den mit einer Kamera ausgestatteten Endoskopiekopf in bestimmte Richtungen zu drehen, um z.B. die Schleimhaut in einem Abschnitt des Magen-Darmtrakts vollständig zu sehen. Dies ist mit heutigen Katheterendoskopen nur bedingt möglich, weil die Katheterspitze nur eingeschränkt beweglich ist. Darüber hinaus weisen übliche Katheterendoskope den Nachteil auf, dass entfernt liegende innere Organe nur schwer oder gar nicht erreichbar sind.

Die passive, durch die natürliche Peristaltik des Magen-Darmtraktes bewegte Endoskopiekapsel hat die genannten Nachteile der Katheterendoskope nicht, ist allerdings auch nicht navigierbar, d.h. die gezielte Sichtung bestimmter Stellen im Inneren das Magen-Darm-Traktes ist nicht möglich. Es wurden daher magnetische Navigations- bzw. Führungssysteme vorgeschlagen, die eine katheter- bzw. drahtlose Führung von Endoskopiekapseln, die ein magnetisches Dipolmoment enthalten, ermöglichen. Eine katheter- oder drahtlose Führung wird nachstehend auch als "berührungslos" bezeichnet.

Die DE 103 40 925 B3 und die WO 2006/092421 A1 beschreiben jeweils eine Magnetspulenanordnung bestehend aus 14 Einzelspulen zur Navigation einer Endoskopiekapsel, einer Videokapsel oder einer sonstigen Sonde. Die Kapsel ist hierbei mit einem magnetischen Element, bspw. einem Permanent- oder Ferromagneten, ausgestattet. Die Magnetspulenanordnung erzeugt Magnetfeldkomponenten $B_x$, $B_y$, $B_z$ entlang der Achsen x, y, z eines kartesischen Koordinatensystems sowie magnetische Gradientenfelder, die eine berührungslose Führung der Endoskopiekapsel ermöglichen.

Hierbei wird ausgenutzt, dass sich das magnetische Element, d.h. ein Körper mit einem magnetischen Dipolmoment m, parallel zur Richtung des magnetischen Feldes B bestehend aus den Magnetfeldkomponenten $B_x$, $B_y$, $B_z$ in Richtung der Achsen des kartesischen Koordinatensystems auszurichten versucht. Da das magnetische Element fest mit der Endoskopiekapsel verbunden ist, kann so die Orientierung der Kapsel beeinflusst werden. Zusätzlich wirkt ausgelöst durch die magnetischen Gradientenfelder $\partial B_x/\partial x$ etc. eine Kraft $F=G{\cdot}m$ mit einer die Gradientenfelder umfassenden Gradientenmatrix G auf das magnetische Dipolmoment m gemäß

$$F = G \cdot m = \begin{pmatrix} \partial B_x / \partial x & \partial B_x / \partial y & \partial B_x / \partial z \\ \partial B_y / \partial x & \partial B_y / \partial y & \partial B_y / \partial z \\ \partial B_z / \partial x & \partial B_z / \partial y & \partial B_z / \partial z \end{pmatrix} \cdot m \ .$$

Die Kraft F und das magnetische Moment m sind dabei - analog zu Magnetfeld B - dreidimensionale Vektoren mit entsprechenden x-, y- und z-Komponenten. Die 3 x 3 Gradientenmatrix G ist aufgrund der Maxwell-Gleichungen *rot H* = 0 und *div B* = 0 sowie wegen $B = \mu_0 \cdot H$ symmetrisch und spurfrei, d.h. sie enthält mit $\partial B_x/\partial y$ (=$\partial B_y/\partial x$), $\partial B_x/\partial z$ (=$\partial B_z/\partial x$), $\partial B_y/\partial z$ (=$\partial B_z/\partial y$) und zweien der drei Diagonalelemente (bspw. $\partial B_x/\partial x$ und $\partial B_y/\partial y$) fünf unabhängige Gradientenfelder.

Durch eine gezielte Ansteuerung der Einzelspulen der Magnetspulenanordnung können das Magnetfeld B und die Gradientenfelder beliebig eingestellt werden. Es ist damit zum Einen möglich, das magnetische Objekt zu drehen und es somit beliebig in einem Arbeitsraum innerhalb der Magnetspulenanordnung auszurichten. Zum Anderen ist es möglich, eine Kraft F auf das magnetische Objekt auszuüben, um es zusätzlich zur Drehung translatorisch zu verschieben. Hierzu werden acht quasistatische magnetische Freiheitsgrade realisiert, nämlich die Magnetfeldkomponenten $B_x$, $B_y$, $B_z$ sowie zwei der drei Einträge der Diagonalelemente (beispielsweise $\partial B_x/\partial x$ und $\partial B_y/\partial y$) und drei der Nebendiagonalelemente (beispielsweise $\partial B_x/\partial y$, $\partial B_x/\partial x$, $\partial B_z/\partial y$) der Gradientenmatrix G.

Die in DE 103 40 925 B3 und WO 2006/092421 A1 beschriebenen Systeme haben den Nachteil, dass sie aufgrund der dort benötigten 14 einzeln angesteuerten Spulen wegen der hohen Zahl an Spulen und Ansteuereinheiten in der Form von Leistungsverstärkern relativ kostenintensiv in Herstellung und Installation sind.

Die Druckschrift WO 2008/004497 A1 offenbart eine Spulenanordnung zur Führung einer Endoskopiekapsel mit einer kubischen Anordnung von drei Paaren von gegenüberliegenden Einzelspulen. Für ein jeweiliges Paar ist ein gemeinsamer Spulentreiber zur Erzeugung eines Magnetfelds vorgesehen.

Das Dokument WO 2007/110278 A1 offenbart ein Magnetspulensystem zur Fernsteuerung einer Arbeitskapsel aus mehreren, den Arbeitsraum des Systems umgebenden Erregerspulen, deren Magnetfelder zur Navigation der Arbeitskapsel und zur Übertragung von Steuersignalen an die Kapsel genutzt werden.

Aufgabe der Erfindung ist es daher, ein kostengünstigeres magnetisches Führungssystem bestehend aus einer Spulenanordnung und mehreren, den Spulen zugeordneten Ansteuereinheiten anzugeben.

Diese Aufgabe wird durch eine Spulenanordnung gemäß Patentanspruch 1 bzw. eine Spulenanordnung gemäß Patentanspruch 13 bzw. eine Spulenanordnung gemäß Patentanspruch 14 gelöst. Weiterbildungen der Erfindung sind in den abhängigen Ansprüchen definiert.

Die erfindungsgemäße Spulenanordnung dient zur Führung eines magnetischen Objektes mit einem magnetischen Dipolmoment in einem Arbeitsraum, für den ein kartesisches Koordinatensystem mit drei Achsen umfassend eine horizontale erste Achse, eine horizontale zweite Achse und eine vertikale dritte Achse vorgegeben ist. Die Spulenanordnung beinhaltet eine Mehrzahl von Einzelspulen zur Erzeugung von Komponenten eines Magnetfelds und von Gradientenfeldern, wobei die Gradientenfelder durch die eingangs beschriebene Gradientenmatrix G beschrieben werden. Ferner ist eine Mehrzahl von Ansteuereinheiten vorgesehen, welche insbesondere als entsprechende Leistungsverstärker realisiert werden.

Um gemäß einer ersten Ausführungsform der Erfindung eine Bewegung eines magnetischen Objektes in jeder Raumrichtung mit einer geringen Anzahl an Einzelspulen zu erreichen, wird folgende Ausgestaltung und Anordnung von Einzelspulen verwendet:

Die Mehrzahl von Einzelspulen umfasst eine Mehrzahl von ersten Einzelspulen, wobei der Arbeitsraum gesehen aus Richtung der Längsachse der jeweiligen ersten Einzelspule innerhalb des Umfangs der jeweiligen ersten Einzelspule liegt. Hier und im Folgenden bezeichnet die Längsachse einer Einzelspule die Achse, um die sich die Wicklungen der Spule erstrecken. Die Wicklungen bilden dabei den Umfang der Spule. Die Breite einer Einzelspule bezeichnet im Folgenden die Breite der Wicklungen in Richtung der Längsachse.

Erfindungsgemäß ist einer oder mehreren der Achsen des kartesischen Koordinatensystems jeweils zumindest ein erstes Spulenpaar aus zwei ersten Einzelspulen zugeordnet, welche entlang der jeweiligen Achse an gegenüberliegenden Seiten des Arbeitsraums in im Wesentlichen parallelen Ebenen angeordnet sind. Um die Anzahl der Spulen möglichst gering zu halten, ist dabei einer oder mehreren der Achsen des kartesischen Koordinatensystems anstatt eines ersten Spulenpaars eine einzelne erste Einzelspule oder keine erste Einzelspule zugeordnet, wobei der Fall "keine erste Einzelspule" bedeutet, dass entlang der Achse weder ein Spulenpaar aus ersten Einzelspulen noch eine erste Einzelspule vorgesehen ist. Wird anstatt des Spulenpaars eine erste Einzelspule verwendet, ist der Arbeitsraum zumindest teilweise von dem Umfang einer jeweiligen einzelnen ersten Einzelspule umgeben, d.h. die Breite des Umfangs deckt zumindest teilweise den Arbeitsraum ab.

Ferner ist neben den ersten Einzelspulen eine Mehrzahl von zweiten Einzelspulen vorgesehen, wobei der Arbeitsraum in der Richtung der Längsachse einer jeweiligen zweiten Einzelspule außerhalb des Umfangs der jeweiligen zweiten Einzelspule liegt. Dabei sind einer oder mehreren der Achsen des kartesischen Koordinatensystems jeweils zumindest ein zweites Spulenpaar aus zwei zweiten Einzelspulen zugeordnet, welche im Wesentlichen in einer gemeinsamen Ebene entlang der jeweiligen Achse an gegenüberliegenden Seiten des Arbeitsraums angeordnet sind. Hier und im Folgenden ist unter einer gemeinsamen Ebene eine Ebene zu verstehen, welche sich durch den Umfang der zweiten Spulen des jeweiligen Spulenpaars erstreckt. Es handelt sich somit um eine flügelartige Anordnung der entsprechenden zweiten Einzelspulen eines zweiten Spulenpaars in der Umgebung des Arbeitsraums.

Die obige Ausführungsform der erfindungsgemäßen Spulenanordnung hat den Vorteil, dass die Anzahl der verwendeten Einzelspulen dadurch reduziert wird, dass zumindest ein Spulenpaar aus zwei ersten Einzelspulen durch eine einzelne erste Einzelspule ersetzt wird. In einer besonders bevorzugten Variante wird auch ferner die Anzahl der verwendeten Ansteuereinheit dadurch reduziert, dass zumindest zwei Einzelspulen durch eine gemeinsame Ansteuereinheit ansteuerbar sind.

Eine spezielle Ausgestaltung der ersten Ausführungsform der Erfindung umfasst genau elf Einzelspulen. Dabei sind zwei erste Spulenpaare vorgesehen, wobei eines der ersten Spulenpaare der ersten Achse zugeordnet ist und das andere der ersten Spulenpaare der dritten Achse zugeordnet ist. Demgegenüber ist der zweiten Achse eine einzelne erste Einzelspule zugeordnet. Es ist ferner ein zweites Spulenpaar vorgesehen, welches der ersten Achse zugeordnet ist und in einer Ebene angeordnet ist, welche durch die erste und dritte Achse aufgespannt ist. Darüber hinaus sind zwei zweite Spulenpaare vorgesehen, welche der dritten Achse zugeordnet sind und welche in Ebenen im Wesentlichen senkrecht zueinander angeordnet sind, wobei eine der Ebenen durch die erste und dritte Achse aufgespannt ist und die andere der Ebenen durch die zweite und dritte Achse aufgespannt ist. Auf diese Weise kann ein magnetisches Objekt, auf das mit der Spulenanordnung Kräfte ausgeübt werden, mit lediglich elf Spulen beliebig im Raum ausgerichtet werden, und es können beliebige Kräfte auf das magnetische Objekt ausgeübt werden.

In einer alternativen Variante, welche ebenfalls eine beliebige Ausrichtung und Kraftausübung auf das magnetische Objekt ermöglicht, umfasst analog zu der gerade beschriebenen Ausführungsform die gleichen ersten Spulenpaare und die gleiche erste Einzelspule. Ferner ist ebenfalls ein zweites Spulenpaar vorgesehen, welches der ersten Achse zugeordnet ist und in einer Ebene angeordnet ist, welche durch die erste und die dritte Achse aufgespannt ist. Ebenso ist ein zweites Spulenpaar vorgesehen, welches der dritten Achse zugeordnet ist und welches in einer Ebene angeordnet ist, die durch die erste und dritte Achse aufgespannt ist. Im Unterschied zur vorangegangenen Ausführungsform ist ein weiteres zweites Spulenpaar vorgesehen, welches der ersten Achse zugeordnet ist und in einer Ebene angeordnet ist, welche durch die erste und zweite Achse aufgespannt wird.

In einer besonders bevorzugten Variante der beiden im Vorangegangenen beschriebenen speziellen Ausführungsbeispiele wird eine minimale Anzahl von acht Ansteuereinheiten zur Ansteuerung der Spulen verwendet. Dabei werden im Betrieb der Spulenanordnung alle ersten Einzelspulen durch separate Ansteuereinheiten und die zweiten Spulenpaare jeweils durch eine gemeinsame Ansteuereinheit angesteuert.

Eine zweite Ausführungsform der Erfindung dient dazu, ein magnetisches Objekt im Raum beliebig auszurichten und nur Magnetkräfte auf das magnetische Objekt in der Ebene auszuüben, die durch die Richtung des magnetischen Dipolmoments des magnetischen Objektes und die vertikale Achse aufgespannt werden. Diese Variante wird insbesondere im Bereich der Kapselendoskopie für ein Magenscreening verwendet, wie es beispielsweise in der Druckschrift WO 2007/077922 A1 beschrieben ist. Dabei bewegt sich die Endoskopiekapsel im Magen des Patienten in Wasser, welches der Patient zuvor getrunken hat.

In einer Ausgestaltung der zweiten Ausführungsform umfasst die Spulenanordnung genau neun Einzelspulen. Dabei sind zwei erste Spulenpaare vorgesehen, wobei eines der ersten Spulenpaare der ersten Achse zugeordnet ist und das andere der ersten Spulenpaare der dritten Achse zugeordnet ist. Ferner ist eine einzelne erste Einzelspule vorgesehen, welche der zweiten Achse zugeordnet ist. Darüber hinaus beinhaltet die Spulenanordnung zwei zweite Spulenpaare, welche der dritten Achse zugeordnet sind und welche in Ebenen im Wesentlichen senkrecht zueinander angeordnet sind, wobei eine der Ebenen durch die erste und dritte Achse aufgespannt ist und die andere der Ebenen durch die zweite und dritte Achse aufgespannt ist.

In einer alternativen Variante der obigen Ausführungsform mit neun Spulen sind die gleichen ersten Spulenpaare, die gleiche einzelne erste Einzelspule sowie das gleiche, der dritten Achse zugeordnete und in der Ebene der ersten und dritten Achse liegende zweite Spulenpaar vorgesehen. Im Unterschied zur vorangegangenen Ausführungsform ist nunmehr jedoch ein zweites Spulenpaar vorgesehen, welches der ersten Achse zugeordnet ist und in einer Ebene angeordnet ist, welche durch die erste und zweite Achse aufgespannt ist.

In einer besonders bevorzugten Variante der oben beschriebenen Ausgestaltungen der zweiten Ausführungsform werden genau sieben Ansteuereinheiten verwendet, wobei alle ersten Einzelspulen durch separate Ansteuereinheiten ansteuerbar sind und die zweiten Spulenpaare jeweils durch eine gemeinsame Ansteuereinheit ansteuerbar sind.

Eine dritte Ausführungsform der erfindungsgemäßen Spulenanordnung dient dazu, ein magnetisches Objekt beliebig im Raum auszurichten und nur Magnetkräfte in Richtung der Längsachse des magnetischen Objektes darauf auszuüben. Dabei steht das magnetische Dipolmoment des Objekts senkrecht auf der Längsachse des Objekts. Diese Ausführungsform dient bei der Verwendung zur Kapselendoskopie insbesondere dazu, eine Endoskopiekapsel nach Art einer Schlauchnavigation durch den Dünndarm und/oder Dickdarm des zu untersuchenden Patienten zu bewegen.

In einer Variante dieser dritten Ausführungsform umfasst die Spulenanordnung genau acht Einzelspulen. Dabei ist ein erstes Spulenpaar vorgesehen, welches der dritten Achse zugeordnet ist, sowie ein zweites Spulenpaar, welches der ersten Achse zugeordnet ist und in einer Ebene angeordnet ist, welche durch die erste und dritte Achse aufgespannt ist. Ferner sind zwei weitere zweite Spulenpaare vorgesehen, welche der dritten Achse zugeordnet sind und welche in Ebenen im Wesentlichen senkrecht zueinander angeordnet sind, wobei eine der Ebenen durch die erste und dritte Achse aufgespannt ist und die andere der Ebenen durch die zweite und dritte Achse aufgespannt ist.

In einer weiteren Variante der dritten Ausführungsform umfasst die Spulenanordnung aus acht Einzelspulen ein erstes Spulenpaar, welches der ersten Achse zugeordnet ist, ein zweites Spulenpaar, welches der ersten Achse zugeordnet ist und in einer Ebene angeordnet ist, welche durch die erste und dritte Achse aufgespannt ist, sowie ein weiteres zweites Spulenpaar, welches der ersten Achse zugeordnet ist und in einer Ebene angeordnet ist, welche durch die erste und zweite Achse aufgespannt ist. Darüber hinaus ist noch ein weiteres zweites Spulenpaar vorgesehen, welches der dritten Achse zugeordnet ist und in einer Ebene angeordnet ist, welche durch die erste und dritte Achse aufgespannt ist.

In den soeben beschriebenen Varianten der dritten Ausführungsform können genau sieben Ansteuereinheiten vorgesehen sein, wobei alle zweiten Einzelspulen durch separate Ansteuereinheiten ansteuerbar sind und die zwei ersten Einzelspulen des ersten Spulenpaars durch eine gemeinsame Ansteuereinheit ansteuerbar sind. Alternativ kann die Anzahl der Ansteuereinheiten auf sechs vermindert werden, wobei in diesem Fall die zweiten Einzelspulen des zweiten Spulenpaars, welches der ersten Achse zugeordnet ist und in einer Ebene angeordnet ist, welche durch die erste und dritte Achse aufgespannt ist, durch eine gemeinsame Ansteuereinheit ansteuerbar sind. Ferner sind die zwei ersten Einzelspulen des ersten Spulenpaars ebenfalls durch eine gemeinsame Ansteuereinheit ansteuerbar. Alle restlichen Einzelspulen der Spulenanordnung sind in dieser Ausführungsform durch separate Ansteuereinheiten ansteuerbar.

In einer vierten Ausführungsform der erfindungsgemäßen Spulenanordnung soll das magnetische Objekt im Raum beliebig ausrichtbar sein und nur Magnetkräfte in Richtung der Längsachse des magnetischen Objektes ausübbar sein. Dabei ist das magnetische Dipolmoment des magnetischen Elements im magnetischen Objekt vorzugsweise entlang der Längsachse des magnetischen Objektes ausgerichtet, was zur Einschränkung führt, dass - im Gegensatz zur vorangegangenen Ausführungsform - das magnetische Objekt, beispielsweise in der Form einer Kapsel, nicht um seine Längsachse drehbar ist. Diese Ausführungsform kommt vorzugsweise im Bereich der Kapselendoskopie zum Einsatz, und zwar bei der "Schlauchnavigation" von Kapseln durch den Dünndarm bzw. Dickdarm.

In einer ersten Variante der vierten Ausführungsform sind lediglich erste Spulenpaare aus ersten Einzelspulen vorgesehen, wobei die ersten Einzelspulen zumindest eines ersten Spulenpaars durch eine gemeinsame Ansteuereinheit ansteuerbar ist. Bei dieser Variante kann gänzlich auf die flügelartig angeordneten zweiten Spulenpaare verzichtet werden und die Anzahl der Leistungsverstärker wird dadurch vermindert, dass zumindest ein erstes Spulenpaar durch eine gemeinsame Ansteuereinheit angesteuert wird. Es sind dabei sechs erste Einzelspulen vorgesehen, wobei jeder Achse ein erstes Spulenpaar aus zwei ersten Einzelspulen zugeordnet ist. Ferner umfasst diese Variante genau fünf Ansteuereinheiten, wobei die zwei ersten Einzelspulen eines ersten Spulenpaars mit einer gemeinsamen Ansteuereinheit ansteuerbar sind und die restlichen ersten Einzelspulen durch separate Ansteuereinheiten ansteuerbar sind.

In einer weiteren Variante der vierten Ausführungsform der erfindungsgemäßen Spulenanordnung sind wiederum ausschließlich erste Einzelspulen vorgesehen, wobei nunmehr einer oder mehreren der Achsen des kartesischen Koordinatensystems anstatt eines ersten Spulenpaars jeweils eine einzelne erste Einzelspule zugeordnet ist, wobei der Arbeitsraum zumindest teilweise von dem Umfang einer jeweiligen einzelnen ersten Einzelspule umgeben ist. Vorzugsweise sind dabei alle ersten Einzelspulen durch separate Ansteuereinheiten ansteuerbar. In einer bevorzugten Variante umfasst die Spulenanordnung dabei genau fünf erste Einzelspulen, d.h. es sind zwei erste Spulenpaare vorgesehen und genau ein erstes Spulenpaar wird durch eine einzelne erste Einzelspule ersetzt.

In einer bevorzugten Ausgestaltung der oben beschriebenen Ausführungsformen der erfindungsgemäßen Spulenanordnung umfassen die Einzelspulen zumindest teilweise Ringspulen und/oder Sattelspulen. Ebenso können die Einzelspulen zumindest teilweise Flächenspulen umfassen, insbesondere mit einer Breite in Längsrichtung, welche größer als die Dicke der Spule ist, wobei die Breite vorzugsweise zumindest das Zehnfache der Dicke beträgt. Insbesondere kann jede Variante der in der deutschen Patentanmeldung mit der Nummer 10 2008 004 871.2-35 beschriebenen Ausführungsformen von Spulen in der erfindungsgemäßen Anordnung zum Einsatz kommen. Der gesamte Offenbarungsgehalt der soeben genannten deutschen Patentanmeldung wird dabei zum Inhalt der vorliegenden Anmeldung gemacht.

Wie bereits erwähnt, wird die erfindungsgemäße Spulenanordnung vorzugsweise im Bereich der magnetischen Kapselendoskopie eingesetzt. In einer bevorzugten Variante beinhaltet die Spulenanordnung dabei einen Patiententisch, auf dem im Betrieb der Spulenanordnung der zu untersuchende Patient liegt, wobei der Patiententisch derart angeordnet ist, dass sich die Längsachse eines Patienten auf dem Patiententisch im Wesentlichen in Richtung der zweiten Achse des kartesischen Koordinatensystems erstreckt.

Ausführungsbeispiele der Erfindung werden nachfolgend anhand der beigefügten Figuren detailliert beschrieben.

Es zeigen:

Fig. 1          eine schematische Darstellung eines 12-Spulen-Systems mit acht Leistungsverstärkern basierend auf einer früheren Patentanmeldung der Anmelderin;

Fig. 2          eine schematische Darstellung eines 11-Spulen-Systems mit acht Leistungsverstärkern gemäß einer ersten Ausführungsform der Erfindung;

Fig. 3 und Fig. 4    schematische Darstellungen eines 10-Spulen-Systems mit sieben Leistungsverstärkern basierend auf einer früheren Patentanmeldung der Anmelderin;

Fig. 5          ein 9-Spulen-System mit sieben Leistungsverstärkern gemäß einer zweiten Ausführungsform der Erfindung;

Fig. 6 und Fig. 7    ein 8-Spulen-System mit sechs bzw. sieben Leistungsverstärkern gemäß einer dritten Ausführungsform der Erfindung;

Fig. 8 und Fig. 9    ein 6-Spulen-System bzw. ein 5-Spulen-System mit fünf Leistungsverstärkern gemäß einer vierten Ausführungsform der Erfindung;

Fig. 10        eine perspektivische Darstellung eines konkreten Ausführungsbeispiels eines Spulen-Systems gemäß Fig. 3; und

Fig. 11        eine schematische Darstellung des Stromflusses durch das Spulenpaar aus Spulen 9 und 10 der Spulenanordnung gemäß Fig. 10.

Bevor auf die einzelnen Ausführungsformen beschrieben werden, wird zunächst der Zusammenhang zwischen dem magnetischen Dipolmoment eines mit der erfindungsgemäßen Spulenanordnung geführten Objekts und dem Stromfluss bzw. dem entsprechend generierten Magnetfeld des erfindungsgemäßen Spulen-Systems erläutert. Als magnetisches Objekt wird vorzugsweise eine Endoskopiekapsel verwendet, die ein magnetisches Element, z.B. einen Permanentmagneten enthält und die im Inneren eines Patienten (nicht dargestellt) positioniert wird, beispielsweise indem der Patient die Kapsel schluckt. Eine solche Kapsel umfasst üblicherweise eine Kamera zur Aufnahme von Bildern von inneren Organen des Patienten sowie ein entsprechendes Sendemodul, mit dem die aufgenommenen Bilder an eine Verarbeitungseinheit mit einem entsprechenden Empfänger gesendet werden. Im Folgenden wird das magnetische Dipolmoment mit m bezeichnet; es stellt einen dreidimensionalen Vektor dar. Das Dipolmoment wird dabei z.B. mit Hilfe eines Permanentmagneten generiert und kann in der Kapsel in Richtung der Kapsellängsachse bzw. gegebenenfalls auch senkrecht zur Kapsellängsachse ausgerichtet sein. Der Permanentmagnet ist fest mit der Endoskopiekapsel verbunden, so dass Kräfte und Drehmomente am Permanentmagneten, die durch die erfindungsgemäße Spulenanordnung erzeugt werden, direkt auf die Endoskopiekapsel übertragen werden.

Allgemein wird davon ausgegangen, dass in dem Spulen-System n Spulen vorgesehen sind, in welchen die Ströme $I_1$ bis $I_n$ fließen. In Vektorschreibweise lauten das magnetische Moment m bzw. der Spulenstromvektor I wie folgt:

$$m = \begin{pmatrix} m_x \\ m_y \\ m_z \end{pmatrix}$$

$$I = \begin{pmatrix} I_1 \\ \vdots \\ I_n \end{pmatrix}$$

$m_x$, $m_y$ bzw. $m_z$ stellen dabei die Komponenten des magnetischen Dipolmoments in Richtung der x- bzw. y- bzw. z-Achse des dem Spulen-System zugeordneten kartesischen Koordinatensystems dar.

Durch die Spulenströme wird ein Magnetfeld erzeugt, welches ein Drehmoment auf die Endoskopiekapsel ausübt, so dass sich die Endoskopiekapsel in Richtung der Feldlinien des magnetischen Feldes ausrichtet. Ferner ergibt sich eine magnetische Kraft auf die Endoskopiekapsel durch den Gradienten des mittels des Spulensystems erzeugten Magnetfeldes. Diese Kraft wird im Folgenden als F bezeichnet und lautet in Vektorschreibweise wie folgt:

$$F = \begin{pmatrix} F_x \\ F_y \\ F_z \end{pmatrix}$$

Dabei stellen $F_x$, $F_y$ und $F_z$ wiederum die entsprechenden Komponenten der Kraft in x- bzw. y- bzw. z-Richtung dar.

In Matrixschreibweise ergibt sich folgender Zusammenhang zwischen dem durch das Spulen-System am Ort r generierten Magnetfeld B sowie der Kraft F einerseits und dem Spulenstromvektor I andererseits:

$$\begin{pmatrix} B \\ F \end{pmatrix} = \begin{pmatrix} U_1 & 0 \\ 0 & U_2 \end{pmatrix} \begin{pmatrix} V_1 \\ V_2 \end{pmatrix} I$$

Dabei gilt:

$$U_1 = \begin{pmatrix} 1 & 0 & 0 \\ 0 & 1 & 0 \\ 0 & 0 & 1 \end{pmatrix}$$

$$V_1 \cdot I = \begin{pmatrix} B_x \\ B_y \\ B_z \end{pmatrix},$$

$$V_2 \cdot I = \begin{pmatrix} \partial B_x / \partial x \\ \partial B_y / \partial x \\ \partial B_z / \partial x \\ \partial B_z / \partial y \\ \partial B_y / \partial y \end{pmatrix}$$

$V_1$ und $V_2$ sind dabei Matrizen, welche durch den Ort r sowie die konkrete Ausführungsform des Spulen-Systems vorgegeben sind.

Man erkennt, dass die magnetische Kraft F nur von fünf Gradientenfeldern $\partial B_x/\partial x$, $\partial B_y/\partial x$, $\partial B_z/\partial z$, $\partial B_z/\partial y$ und $\partial B_y/\partial y$ abhängt, was sich aus der eingangs erläuterten Tatsache ergibt, dass die Gradientenmatrix aufgrund der Maxwellgleichungen symmetrisch und spurfrei ist.

Basierend auf der Gleichung $F=G \cdot m$ ergeben sich folgende Werte für die Matrix $U_2$:

$$U_2 = \begin{pmatrix} m_x & m_y & m_z & 0 & 0 \\ 0 & m_x & 0 & m_z & m_y \\ -m_z & 0 & m_x & m_y & -m_z \end{pmatrix}$$

*Spaltennr.* $\rightarrow$ 1 2 3 4 5

Bei der Bewegung einer Endoskopiekapsel sind dabei verschiedene Szenarien von vorgebbaren Bewegungsrichtungen und Ausrichtungen des Dipolmoments denkbar, welche für verschiedene medizinische Anwendungen relevant sind. Hieraus ergeben sich unterschiedliche Konfigurationen von Spulensystemen und entsprechenden Leistungsverstärkern zur Ansteuerung der Spulen-systeme. Aufgrund vorgegebener Randbedingungen können dabei in bestimmten Szenarien Paare von Spulen mit dem gleichen Leistungsverstärker angesteuert werden und bestimmte Spulenpaare weggelassen bzw. durch eine einzelne Spule ersetzt werden. Nachfolgend werden Ausführungsformen beschrieben, mit denen in Abhängigkeit von verschiedenen Anforderungen minimale Spulensystem-Konfigurationen, d.h. Konfigurationen mit einer möglichst geringen Anzahl an Spulen und Leistungsverstärkern, erreicht werden.

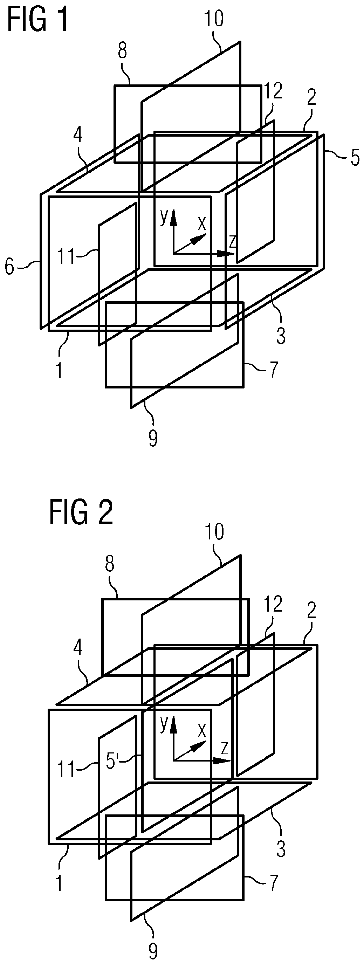

Anhand von Fig. 1 bis Fig. 3 werden zunächst Spulensystem-Konfigurationen erläutert, mit denen eine Endoskopiekapsel im Raum beliebig ausrichtbar sein soll und eine Magnetkraft in beliebigen Raumrichtungen auf die Kapsel ausübbar sein soll. Diese Navigationsanforderungen werden als Fall (A) bezeichnet. Wie oben dargelegt, hängt die magnetische Kraft F linear vom magnetischen Dipolmoment m der Kapsel ab. Es genügt deshalb für den nachfolgenden Fall (A) und auch alle weiteren Fälle (B) bis (D) die folgenden Szenarien zu analysieren:

(X) das Dipolmoment ist in x-Richtung ausgerichtet,
(Y) das Dipolmoment ist in y-Richtung ausgerichtet,
(Z) das Dipolmoment ist in z-Richtung ausgerichtet.

Gemäß dem Fall (A.X), bei dem das Dipolmoment nur die x-Komponente $m_x$ aufweist, sind nur die Spalten mit den Nummern 1, 2 und 3 der obigen Matrix $U_2$ relevant, welche mit den Feldgradienten $\partial B_x/\partial x$, $\partial B_y/\partial y$ und $\partial B_z/\partial_x$ kombiniert werden. Für den Fall (A.Y) sind aufgrund des Dipolmoments $m_y$ nur die Spalten 2, 4 und 5 der obigen Matrix

$U_2$ relevant. Für den Fall (A.Z), bei dem lediglich ein Dipolmoment mit der Komponente $m_z$ vorliegt, spielen nur die Spalten mit den Nummern 3, 4 und 5 der Matrix $U_2$ eine Rolle. Aus der Kombination aller obiger Fälle ergibt sich, dass für den Fall (A) alle fünf Feldgradienten in Verbindung mit den drei magnetischen Grundfeld-Komponenten benötigt werden, d.h. es werden mindestens acht Leistungsverstärker zur Ansteuerung der Spulen des Spulen-Systems benötigt.

Fig. 1 zeigt in schematischer Darstellung ein entsprechendes 12-Spulen-System mit acht Leistungsverstärkern, dessen Aufbau auf die deutsche Patentanmeldung mit der Nr. 10 2008 004 871.2-35 zurückgeht. Alle in dieser Patentanmeldung dargestellten Ausführungsformen von verwendbaren Spulen können auch in den erfindungsgemäßen Varianten der nachfolgend beschriebenen Spulen-Systeme eingesetzt werden. Das in Fig. 1 gezeigte Spulen-System umfasst die zwölf Einzelspulen 1 bis 12, wobei analog zu der oben genannten früheren deutschen Patentanmeldung die erste bis vierte Spule 1 bis 4 als identisch geformte Sattelspulen ausgebildet sein können, welche einen Arbeitsraum umgeben, der im Bereich des dargestellten kartesischen Koordinatensystems mit der horizontalen x- bzw. z-Achse und der vertikalen y-Achse angeordnet ist.

Bei der Verwendung des Spulen-Systems zur Kapselendoskopie ist in Fig. 1 und auch in den Ausführungsformen aller anderen Figuren bei der Durchführung der endoskopischen Untersuchung ein Körperabschnitt des Patienten von den Spulen 1 bis 4 umgeben, d.h. die Längsachse des Patienten verläuft parallel zu der z-Achse. Der Körper des Patienten erstreckt sich somit durch das Innere der weiteren Spulen 5 und 6, welche analog zu der oben genannten früheren deutschen Patentanmeldung als Ringspulen ausgestaltet sein können. Im Arbeitsraum erfolgt dabei die Bewegung des entsprechenden magnetischen Objekts bzw. der Endoskopiekapsel, d.h. der Arbeitsraum liegt im Inneren des Patientenkörpers. Anstatt der Verwendung von Sattelspulen 1 bis 4 bzw. Ringspulen 5 und 6 können auch Flächenspulen eingesetzt werden, welche im Unterschied zu Sattelspulen eben ausgebildet sind und vorzugsweise einen rechteckigen Querschnitt aufweisen. Die Flächenspulen sind dabei vorzugsweise als breite Flächenspulen ausgebildet, deren Wickelpaket breiter als hoch ist, d.h. die Breite entlang der Längsachse der Spule ist wesentlich größer ist als die Dicke bzw. die Wickelhöhe der Spule.

Wie sich aus Fig. 1 ergibt, umgeben die Spulen 1 bis 6 den Arbeitsraum, dessen Ursprung durch den Ursprung des dargestellten Koordinatensystems wiedergegeben ist. Der Arbeitsraum ist dabei zentriert im durch die Spulen 1 bis 6 gebildeten Volumen angeordnet und er stellt einen Quader dar, dessen gegenüberliegenden Seiten in etwa den halben Abstand wie die entsprechenden gegenüberliegenden Spulen aufweisen. Die Spulen 1 bis 6 stellen somit erste Einzelspulen im Sinne der Ansprüche dar, bei denen der Arbeitsraum gesehen aus der Richtung der Längsachse einer jeweiligen Einzelspule innerhalb des Umfangs der jeweiligen Spule liegt. Entsprechend gegenüber liegende Spulen 1 und 2 bzw. 3 und 4 bzw. 5 und 6 stellen dabei erste Spulenpaare dar, denen eine entsprechende Achse des kartesischen Koordinatensystems zugeordnet ist, wobei die einzelnen Spulen des Spulenpaars an gegenüberliegenden Seiten des Arbeitsraums in im Wesentlichen parallelen Ebenen angeordnet sind. Das Spulenpaar aus Spulen 1 und 2 dient dabei zur Erzeugung der Magnetfeldkomponente $B_x$ des generierten Magnetfelds B, wobei bei getrennter Ansteuerung der beiden Spulen mit jeweils einem Leistungsverstärker ferner das Gradientenfeld $\partial B_x/\partial x$ erzeugbar ist. Die Spulen 3 und 4 dienen zur Einstellung der Magnetfeldkomponente $B_y$, wobei ferner das Gradientenfeld $\partial B_y/\partial y$ erzeugbar ist, falls die beiden Spulen durch separate Leistungsverstärker angesteuert werden. Die Spulen 5 und 6 dienen zur Erzeugung der Magnetfeldkomponente $B_z$, wobei ferner das Gradientenfeld $\partial B_z/\partial z$ erzeugbar ist, sofern die beiden Spulen des Spulenpaars durch separate Leistungsverstärker angesteuert werden. Sofern in allen weiteren Ausführungsformen auf entsprechende Spulen 1 bis 6 verwiesen wird, so sind diese Spulen analog zu Fig. 1 angeordnet und haben auch die gleiche Funktion der Erzeugung einer Magnetfeldkomponente bzw. eines Gradientenfelds.

Um ferner die Nebendiagonalelemente der eingangs beschriebenen Gradientenmatrix G zu erzeugen, sind in dem Beispiel der Fig. 1 ferner Spulen 8 bis 12 vorgesehen, welche zweite Einzelspulen im Sinne der Ansprüche darstellen. Die Spulen 7 und 8 bilden dabei ein Spulenpaar, dessen beide Spulen versetzt vom Arbeitsraum entlang der y-Achse in der y-z-Ebene angeordnet sind. Ebenso bilden die Spulen 9 und 10 ein Spulenpaar, welches entlang der y-Achse versetzt um den Arbeitsraum angeordnet ist, jedoch gegenüber dem Spulenpaar aus Spulen 7 und 8 um 90° verdreht in der x-y-Ebene angeordnet ist. Ferner ist ein Spulenpaar aus Spulen 11 und 12 vorgesehen, wobei die Spulen versetzt zueinander entlang der x-Achse in der x-y-Ebene an gegenüberliegenden Seiten des Arbeitsraums angeordnet sind. In Übereinstimmung mit der Definition der zweiten Einzelspulen gemäß den Patentansprüchen liegt der Arbeitsraum gesehen aus der Richtung der Längsachse einer jeweiligen Spule der Einzelspulen 7 bis 12 außerhalb des Umfangs der jeweiligen Spule.

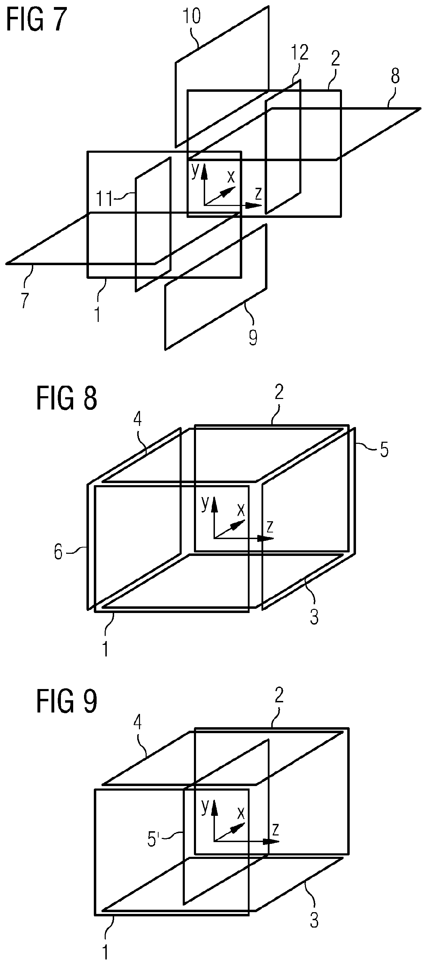

Zur Erzeugung der entsprechenden Gradientenfelder gemäß den Nebendiagonalelementen der Gradientenmatrix G können die einzelnen Spulen 7, 8 bzw. 9, 10 bzw. 11, 12 der jeweiligen Spulenpaare mit einem gemeinsamen Leistungsverstärker angesteuert werden. Das Spulenpaar aus Einzelspulen 7 und 8 dient dabei zur Erzeugung des Gradientenfelds $\partial B_y/\partial x(=\partial B_x/\partial y)$, das Spulenpaar aus Einzelspulen 9, 10 erzeugt das Gradientenfeld $\partial B_z/\partial y(=\partial B_y/\partial z$ und das Spulenpaar aus Einzelspulen 11 und 12 dient zur Erzeugung des Gradientenfelds $\partial B_x/\partial z(=\partial B_z/\partial x)$. Zur Erzeugung des Gradientenfelds $\partial B_x/\partial y$ kann das Spulenpaar 7 und 8 gegebenenfalls auch so angeordnet sein, wie in Fig. 7 wiedergegeben ist, bei der die beiden Spulen 7 und 8 entlang der x-Achse in der x-y-Ebene angeordnet sind. Sofern hier und in den nachfolgenden Ausführungsformen auf die Spulen 7 bis 12 verwiesen wird, besteht die gleiche oben beschriebene Zuordnung der entsprechenden Spulennummern zu der Erzeugung der Gradientenfelder. Im Regelfall sind die einzelnen Spulen dabei auch in der gleichen geometrischen Lage in Bezug auf das Koordinatensystem wie in Fig. 1 angeordnet. Lediglich für die Spulen 7 und 8 existieren zwei alternative Anordnungen zur Erzeugung des Gradientenfeldes $\partial B_x/\partial y$.

Wie bereits erwähnt, werden acht Leistungsverstärker zur Erzeugung der drei Magnetfeldkomponenten $B_x$, $B_y$ und $B_z$ sowie von fünf Gradientenfeldern benötigt, um gemäß dem obigen Fall (A) ein magnetisches Objekt beliebig im Arbeitsraum zu bewegen bzw. auszurichten. In einer ersten Variante werden dabei entsprechende Leistungsverstärker mit den Nummern 1 bis 8 den Spulen 1 bis 12 wie folgt zugeordnet:

Leistungsverstärker 1: Spule 1
Leistungsverstärker 2: Spule 2
Leistungsverstärker 3: Spule 3
Leistungsverstärker 4: Spule 4
Leistungsverstärker 5: Spulen 5 und 6
Leistungsverstärker 6: Spulen 9 und 10
Leistungsverstärker 7: Spulen 7 und 8
Leistungsverstärker 8: Spulen 11 und 12

Analog kann in einer weiteren Variante die Zuordnung zwischen Leistungsverstärker und Spulen wie folgt ausgestaltet sein:

Leistungsverstärker 1: Spulen 1 und 2
Leistungsverstärker 2: Spule 3
Leistungsverstärker 4: Spule 6
Leistungsverstärker 5: Spule 5
Leistungsverstärker 6: Spulen 9 und 10
Leistungsverstärker 7: Spulen 7 und 8
Leistungsverstärker 8: Spulen 11 und 12

Analog besteht auch die Möglichkeit, dass aus der Menge der Spulen 1 bis 6 die Spulen 3 und 4 mit einem gemeinsamen Leistungsverstärker und die restlichen dieser Spulen mit separatem Leistungsverstärker betrieben werden, wobei die Spulen 7 bis 12 in gleicher Weise wie oben beschrieben durch Leistungsverstärker angesteuert werden.
Fig. 2 zeigt eine Variante des Spulensystems der Fig. 1, wobei dieses Spulen-System eine erste Ausführungsform einer erfindungsgemäßen Spulenanordnung darstellt. Es wurde dabei erkannt, dass dasjenige Spulenpaar aus ersten Einzelspulen, welches gemäß den Varianten der Ausführungsform der Fig. 1 mit einem gemeinsamen Leistungsverstärker betrieben wird, durch eine einzelne Spule ersetzt werden kann. Demzufolge wird in der Ausführungsform der Fig. 2 anstatt des Spulenpaars aus Spulen 5 und 6 eine einzige Einzelspule 5' verwendet, welche mittig zwischen den beiden (nunmehr weggelassenen) Spulen 5 und 6 angeordnet ist. Die Einzelspule 5' ist dabei vorzugsweise eine breite Flächenspule, deren Wicklungsdicke wesentlich geringer ist als deren Breite in z-Richtung. Gemäß der Spulenanordnung nach Fig. 2 kann somit die Anzahl der verwendeten Spulen von 12 auf 11 reduziert werden, wodurch die Kosten für das Spulen-System vermindert werden. Die Zuordnung der einzelnen Leistungsverstärker 1 bis 8 zu den entsprechenden Spulen des Spulen-Systems der Fig. 2 ist dabei wie folgt:

Leistungsverstärker 1: Spule 1
Leistungsverstärker 2: Spule 2
Leistungsverstärker 3: Spule 3
Leistungsverstärker 4: Spule 4
Leistungsverstärker 5: Spule 5'
Leistungsverstärker 6: Spulen 9 und 10
Leistungsverstärker 7: Spulen 7 und 8
Leistungsverstärker 8: Spulen 11 und 12

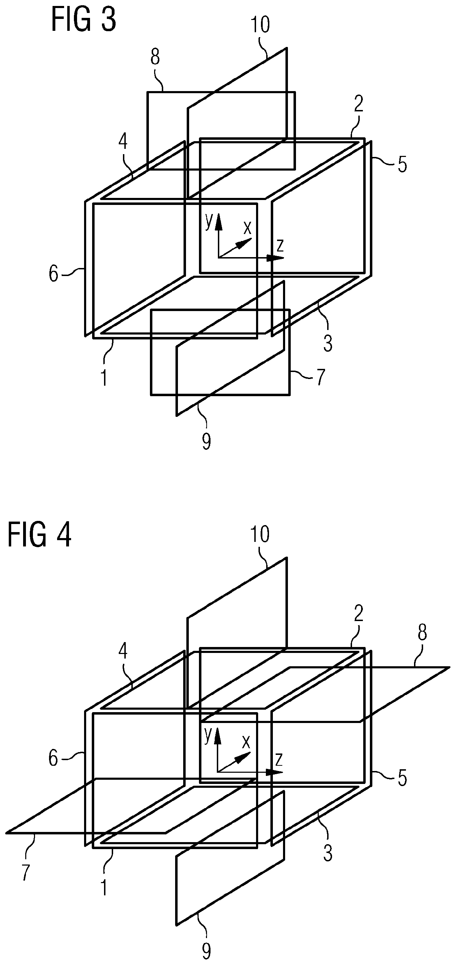

Nachfolgend werden anhand von Fig. 3 bis Fig. 5 Ausführungsformen beschrieben, welche in Kombination mit einem magnetischen Objekt eingesetzt werden, welches beliebig im Raum ausrichtbar sein soll, wobei jedoch nur Magnetkräfte in der Ebene erzeugt werden sollen, die durch das magnetische Dipolmoment des magnetischen Objekts und der vertikalen Achse, d.h. der y-Achse des Spulen-Systems, aufgespannt wird. Dies entspricht im Bereich der Kapselendoskopie beispielsweise dem Fall, dass sich die Kapsel im Magen des Patienten in Wasser bewegt, wobei der Patient bei dieser Untersuchung zuvor eine größere Menge an Wasser getrunken hat. Die in Wasser auf die Kapsel wirkende Auftriebskraft bewirkt dabei, dass die Kapsel an der Wasseroberfläche mit gar keiner oder mit geringer magnetischer Krafteinwirkung schwimmt. Dieser Anwendungsfall wird im Folgenden als Fall (B) bezeichnet. Für den Fall (B.X), bei dem das Dipolmoment nur die Komponente $m_x$ aufweist, muss die Kraft in der x-y-Ebene liegen. Dies bedeutet, dass keine Kraftkomponente $F_z$ generiert werden muss und nur die Spalten 1 und 2 der Matrix $U_2$ mit den Feldgradienten $\partial B_x/\partial x$ und $\partial B_y/\partial x$ kombiniert werden. Für den Fall (B.Y), bei dem das Dipolmoment in y-Richtung ausgerichtet ist, muss die Kraft in y-Richtung ausgerichtet sein. Das heißt, für die Erzeugung der Kraft ist lediglich die Spalte 5 der Matrix $U_2$ relevant. Für den Fall (B.Z), bei dem das Dipolmoment der Kapsel in z-Richtung ausgerichtet ist, muss die Kraft in der y-z-Ebene liegen. In diesem Fall wird keine Kraftkomponente $F_x$ generiert und es sind nur die Spalten mit den Nummern 4 und 5 der Matrix $U_2$ für die Kraftgenerierung relevant. Zusammenfassend sind nur Einträge in den Spalten 1, 2, 4 und 5 für den Fall (B) relevant, was bedeutet, dass nur die Feldgradienten $\partial B_x/\partial x$, $\partial B_y/\partial x$, $\partial B_z/\partial y$ und $\partial B_y/\partial y$, jedoch nicht $\partial B_z/\partial x$, benötigt werden. In Verbindung mit den drei magnetischen Grundfeld-Komponenten sind somit sieben Leistungsverstärker zur Ansteuerung der Spulenanordnung ausreichend.

Fig. 3 und Fig. 4 zeigen jeweils zwei Ausführungsbeispiele von Spulen-Systemen zur Erzeugung von Magnetfeldern für die Navigationsanforderungen gemäß obigem Fall (B). Die gezeigten Beispiele entsprechend im Wesentlichen Ausführungsformen, welche in der oben genannten früheren deutschen Patentanmeldung mit der Nr. 10 2008 004 871.2-35 beschrieben sind. Die dargestellten Spulen-Systeme umfassen nunmehr lediglich zehn Spulen, denn die in den Ausführungsformen der Fig. 1 und Fig. 2 gezeigten Spulen 11 und 12 sind nicht mehr erforderlich, da diese Spulen zur Erzeugung des Feldgradienten $\partial B_z/\partial x$ dienen, der gemäß Fall (B) nicht gesteuert werden muss. Die geometrische Anordnung der Spulen der Fig. 3 entspricht somit der Anordnung gemäß Fig. 1, wobei jedoch die Spulen 9 und 10 weggelassen worden sind. Auch die Zuordnung der Leistungsverstärker zu den Spulen kann gemäß den beiden, in Bezug auf Fig. 1 beschriebenen Varianten erfolgen, wobei lediglich der Leistungsverstärker mit der Nr. 8 zur Ansteuerung des Spulenpaars aus Spulen 11 und 12 weggelassen wird.

Fig. 4 zeigt eine Abwandlung des Spulen-Systems der Fig. 3, welche ebenfalls auf die oben genannte frühere deutsche Patentanmeldung zurückgeht. Der einzige Unterschied dieser Ausführungsform gegenüber der Ausführungsform der Fig. 3 besteht darin, dass nunmehr die Spulen 7 und 8 anders angeordnet sind. Diese Spulen liegen nunmehr in der x-z-Ebene und sind entlang der x-Achse an gegenüberliegenden Seiten des Arbeitsraums angeordnet. Mit dieser Spulenanordnung wird der gleiche Effekt wie mit der Anordnung der Spulen 7 und 8 in Fig. 3 erreicht, d.h. auch diese Spulen dienen zur Einstellung der Magnetfeldkomponente $\partial B_x/\partial y$. Die Ansteuerung der einzelnen Spulen der Fig. 4 kann analog basierend auf den Zuordnungen von Leistungsverstärkern zu Spulen erfolgen, wie in Bezug auf Fig. 2 beschrieben ist.

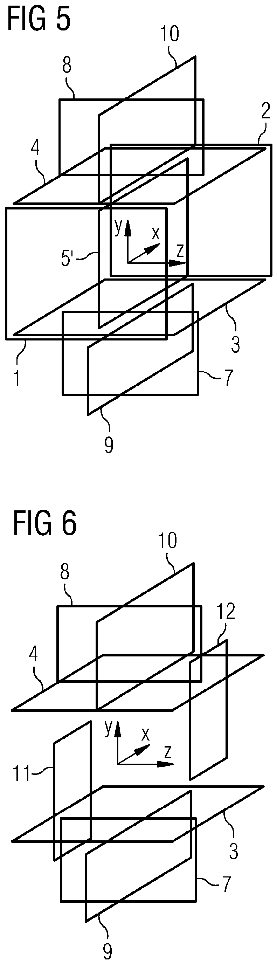

Fig. 5 zeigt eine zweite Ausführungsform eines erfindungsgemäßen Spulen-Systems, mit dem analog zu den Ausführungsformen der Fig. 3 und Fig. 4 eine Navigation der Endoskopiekapsel basierend auf dem Fall (B) ermöglicht wird. Die Ausführungsform gemäß Fig. 5 beruht wiederum auf der Erkenntnis, dass dasjenige Spulenpaar der Spulenpaare aus Spulen 1, 2 bzw. 3, 4 bzw. 5, 6, welches durch einen einzelnen Leistungsverstärker angesteuert wird, durch eine einzelne Spule ersetzt werden kann. In Fig. 5 ist dabei die Variante gezeigt, bei der die Spulen 5 und 6 der Ausführungsform der Fig. 3 durch eine einzelne Spule 5' ersetzt werden. Die restlichen Spulen sind genauso angeordnet, wie dies in der Ausführungsform der Fig. 3 gezeigt ist. Das Spulen-System umfasst nunmehr jedoch nur noch neun Spulen, wobei für die Ausführungsform der Fig. 5 sieben Leistungsverstärker verwendet werden, welche den Spulen wie folgt zugeordnet sind:

Leistungsverstärker 1: Spule 1
Leistungsverstärker 2: Spule 2
Leistungsverstärker 3: Spule 3
Leistungsverstärker 4: Spule 4
Leistungsverstärker 5: Spule 5'
Leistungsverstärker 6: Spulen 9 und 10
Leistungsverstärker 7: Spulen 7 und 8

In einer Abwandlung der Ausführungsform der Fig. 5 können auch entweder das Spulenpaar aus Spulen 1 und 2 oder das Spulenpaar aus Spulen 3 und 4 durch eine einzelne Spule ersetzt werden, wobei in diesem Fall entlang der y-Achse das Spulenpaar aus Spulen 5 und 6 erhalten bleibt. In Analogie zu der Ausführungsform der Fig. 5 werden dabei alle Spulen, welche den ersten Einzelspulen im Sinne von Anspruch 1 entsprechen, durch einen einzelnen Leistungsverstärker angesteuert, wohingegen die Spulen 7, 8 und 9, 10 jeweils durch einen gemeinsamen Leistungsverstärker angesteuert werden.

Fig. 6 und Fig. 7 betreffen erfindungsgemäße Ausführungsformen, gemäß denen ein magnetisches Objekt im Raum beliebig ausrichtbar sein soll, wobei nur Magnetkräfte in Richtung der Längsachse der Kapsel ausübbar sein sollen, und wobei das magnetische Moment des Permanentmagneten in der Kapsel senkrecht zur Kapsellängsachse ausgerichtet ist. Diese Navigationsanforderung, welche nachfolgend als Fall (C) bezeichnet ist, wird insbesondere zur "Schlauchnavigation" von Endoskopiekapseln im Dünndarm bzw. Dickdarm eingesetzt. Aufgrund der Ausrichtung des magnetischen Moments senkrecht zur Kapsellängsachse kann dabei eine Drehung der Kapsel um ihre Längsachse generiert werden, wodurch beispielsweise bei spiralförmigen Kapseln mittels einer entsprechenden Rotationsbewegung eine gute Vorwärtsbewegung der Kapsel durch den Darm ermöglicht wird. Insbesondere können die nachfolgenden Spulen-Konfigurationen gemäß den Ausführungsformen der Fig. 6 und 7 für spiralförmige Kapseln verwendet werden, welche in den Druckschriften US 2003/0020810 A1 bzw. US 2003/0181788 A1 offenbart sind.

Für den Fall (C.X) eines in x-Richtung ausgerichteten Dipolmoments $m_x$ muss die generierte Kraft in der y-z-Ebene liegen. Dies bedeutet, dass keine Kraftkomponente $F_x$ auftritt und nur Einträge in den Spalten mit den Nummern 2 und 3 der Matrix $U_2$ relevant sind, welche mit entsprechenden Feldgradienten $\partial B_y/\partial x$ und $\partial B_z/\partial x$ multipliziert werden. Für den Fall eines in y-Richtung ausgerichteten Dipolmoments $m_y$, welcher dem Fall (C.Y) entspricht, muss die Kraft in der x-z-Ebene liegen. Es tritt dann keine Kraftkomponente $F_y$ auf, so dass von der Matrix $U_2$ lediglich Einträge in den Spalten 2 und 4 relevant sind. Für den Fall (C.Z), bei dem das magnetische Dipolmoment in die z-Richtung gerichtet ist, muss die Kraft in der x-y-Ebene liegen. Das heißt, es tritt keine $F_z$-Kraftkomponente auf und es sind lediglich die Spalten 3 und 4 der Matrix $U_2$ relevant. Insgesamt werden somit für den Fall (C) nur drei Feldgradienten benötigt, nämlich $\partial B_y/\partial x$, $\partial B_z/\partial x$ und $\partial B_z/\partial y$. In Verbindung mit den drei magnetischen Grundfeld-Komponenten werden in diesem Fall mindestens sechs Leistungsverstärker benötigt.

Fig. 6 zeigt eine Ausführungsform einer erfindungsgemäßen Spulenanordnung zur Erfüllung der Navigationsanforderungen gemäß Fall (C). Der Aufbau der Ausführungsform der Fig. 6 ähnelt dabei dem Aufbau der Ausführungsform gemäß Fig. 1, wobei nunmehr jedoch die Spulen 1, 2 und 5, 6 weggelassen worden sind. Zur Generierung der drei Feldgradienten $\partial B_y/\partial x$, $\partial B_z/\partial x$ und $\partial B_z/\partial y$ sowie der drei magnetischen Grundfeld-Komponenten kann eine Speisung mit sechs oder sieben Leistungsverstärkern erfolgen. Im Falle, dass sieben Leistungsverstärker verwendet werden, sind entsprechende Leistungsverstärker mit den Nummern 1 bis 7 den Spulen wie folgt zugeordnet:

    Leistungsverstärker 1: Spule 9
    Leistungsverstärker 2: Spule 10
    Leistungsverstärker 3: Spule 7
    Leistungsverstärker 4: Spule 8
    Leistungsverstärker 5: Spule 11
    Leistungsverstärker 6: Spule 12
    Leistungsverstärker 7: Spulen 3 und 4

In einer Variante, bei der lediglich sechs Leistungsverstärker zur Ansteuerung des 8-Spulen-Systems gemäß Fig. 6 eingesetzt werden, erfolgt die Zuordnung zwischen den Leistungsverstärkern und den Spulen wie folgt:

    Leistungsverstärker 1: Spule 9
    Leistungsverstärker 2: Spule 10
    Leistungsverstärker 3: Spule 7
    Leistungsverstärker 4: Spule 8
    Leistungsverstärker 5: Spulen 11 und 12
    Leistungsverstärker 6: Spulen 3 und 4

Fig. 7 zeigt eine weitere Variante eines 8-Spulen-Systems zur Erfüllung der Navigationsanforderungen gemäß obigem Fall (C).

Die Ausführungsform gemäß Fig. 7 unterscheidet sich von der Ausführungsform gemäß Fig. 6 dahingehend, dass die Spulen 7 und 8, welche zur Generierung der Magnetfeldkomponente $\partial B_y/\partial x$ verwendet werden, nunmehr entlang der x-Achse in der x-z-Ebene angeordnet sind. Ferner wird anstatt des Spulenpaars 3, 4 das Spulenpaar 1, 2 verwendet, welches an gegenüberliegenden Seiten des Arbeitsraums entlang der x-Achse in parallelen Ebenen angeordnet ist.

Zur Ansteuerung der Spulen können wiederum sechs bzw. sieben Leistungsverstärker verwendet werden. In der Variante mit sieben Leistungsverstärkern sind diese Leistungsverstärker den Spulen wie folgt zugeordnet:

    Leistungsverstärker 1: Spule 9
    Leistungsverstärker 2: Spule 10
    Leistungsverstärker 3: Spule 7
    Leistungsverstärker 4: Spule 8
    Leistungsverstärker 5: Spule 11
    Leistungsverstärker 6: Spule 12
    Leistungsverstärker 7: Spulen 1 und 2

In einer Variante, bei der lediglich sechs Leistungsverstärker zur Ansteuerung der Spulen verwendet werden, sind die Leistungsverstärker mit den Nummern 1 bis 6 den Spulen wie folgt zugeordnet:

> Leistungsverstärker 1: Spule 9
> Leistungsverstärker 2: Spule 10
> Leistungsverstärker 3: Spule 7
> Leistungsverstärker 4: Spule 8
> Leistungsverstärker 5: Spulen 11 und 12
> Leistungsverstärker 6: Spulen 1 und 2

Gemäß einer weiteren Navigationsanforderung, welche im Folgenden als Fall (D) bezeichnet wird, soll eine magnetische Kapsel im Raum beliebig ausrichtbar sein und nur Magnetkräfte in Richtung der Kapsellängsachse auf die Kapsel ausübbar sein, wobei das magnetische Moment der Kapsel nunmehr entlang der Kapsellängsachse ausgerichtet ist. Auch dieser Fall (D) betrifft vorzugsweise die Variante der magnetischen Führung einer Endoskopiekapsel für die Schlauchnavigation im Dünn- und/oder Dickdarm. In der Variante (D) kann die Kapsel jedoch aufgrund des parallel zur Längsachse gerichteten magnetischen Dipolmoments nicht um ihre Längsachse gedreht werden.

Für den Fall (D.X) eines in x-Richtung ausgerichteten Dipolmoments $m_x$ wird nur die Kraftkomponente $F_x$ erzeugt, d.h. es ist nur die Spalte 1 der Matrix $U_2$ relevant, welche mit dem Feldgradienten $\partial B_x/\partial x$ kombiniert wird. Für den Fall eines in y-Richtung gerichteten Dipolmoments $m_y$ (Fall (D.Y)) ist die Kraft in y-Richtung ausgerichtet, d.h. es tritt nur die Kraftkomponente $F_y$ auf. In diesem Fall ist nur die Spalte 5 der Matrix $U_2$ relevant, welche mit dem Feldgradienten $\partial B_y/\partial y$ kombiniert wird. Für den Fall eines in z-Richtung ausgerichteten Dipolmoments $m_z$ (Fall (D.Z)) enthält die Kraft nur die Komponente $F_z$, d.h. es sind lediglich die Spalten 1 und 5 der Matrix $U_2$ relevant, welche mit den entsprechenden Feldgradienten $\partial B_x/\partial x$ bzw. $\partial B_y/\partial y$ kombiniert werden. Gemäß Fall (D) müssen somit neben den drei Grundfeld-Komponenten lediglich zwei weitere Diagonalelemente der Gradientenmatrix G generiert werden, denn aufgrund der Spurfreiheit der Gradientenmatrix gilt: $\partial B_z/\partial z = -\partial B_x/\partial x - \partial B_y/\partial y$. Es sind somit fünf Leistungsverstärker zur Realisierung des Falls (D) erforderlich.

Fig. 8 und Fig. 9 zeigen entsprechende Varianten von Spulen-Systemen zur Erfüllung der Navigationsanforderung gemäß Fall (D). Gemäß der Variante nach Fig. 8 werden die sechs Einzelspulen 1 bis 6 verwendet, welche in der Terminologie der Ansprüche den ersten Einzelspulen entsprechen. Da nur fünf Leistungsverstärker benötigt werden, kann eines der Spulenpaare aus Spulen 1 und 2 bzw. 3 und 4 bzw. 5 und 6 durch einen gemeinsamen Leistungsverstärker angesteuert werden. Demzufolge sind folgende Zuordnungen von Leistungsverstärkern mit den Nummern 1 bis 5 zu den entsprechenden Spulen möglich:

> Variante a):

> Leistungsverstärker 1: Spulen 1 und 2
> Leistungsverstärker 2: Spule 3
> Leistungsverstärker 3: Spule 4
> Leistungsverstärker 4: Spule 5
> Leistungsverstärker 5: Spule 6

> Variante b):

> Leistungsverstärker 1: Spule 1
> Leistungsverstärker 2: Spule 2
> Leistungsverstärker 3: Spulen 3 und 4
> Leistungsverstärker 4: Spule 5
> Leistungsverstärker 5: Spule 6

> Variante c):

> Leistungsverstärker 1: Spule 1
> Leistungsverstärker 2: Spule 2
> Leistungsverstärker 3: Spule 3
> Leistungsverstärker 4: Spule 4
> Leistungsverstärker 5: Spulen 5 und 6

Fig. 9 zeigt eine weitere Ausführungsform zur Erfüllung der Navigationsanforderung gemäß Fall (D). Nunmehr wird dasjenige Spulenpaar, welches in der Ausführungsform der Fig. 8 durch einen gemeinsamen Leistungsverstärker angesteuert wird, durch eine einzelne Spule ersetzt. Fig. 9 zeigt dabei die Variante, bei der das Spulenpaar aus Spulen 5 und 6 durch eine Einzelspule 5' ersetzt wird. Ebenso ist es auch möglich, das Spulenpaar aus Spulen 1 und 2 oder das Spulenpaar aus Spulen 3 und 4 durch eine einzelne, jeweils mittig zwischen den wegzulassenden Spulenpaaren angeordnete Spule zu ersetzen. In der Ausführungsform der Fig. 9 werden alle Spulen einzeln über einen separaten Leistungsverstärker gespeist. Das heißt, es besteht folgende Zuordnung zwischen Leistungsverstärkern mit den Nummern 1 bis 5 zu den Spulen:

Leistungsverstärker 1: Spule 1
Leistungsverstärker 2: Spule 2
Leistungsverstärker 3: Spule 3
Leistungsverstärker 4: Spule 4
Leistungsverstärker 5: Spule 5'

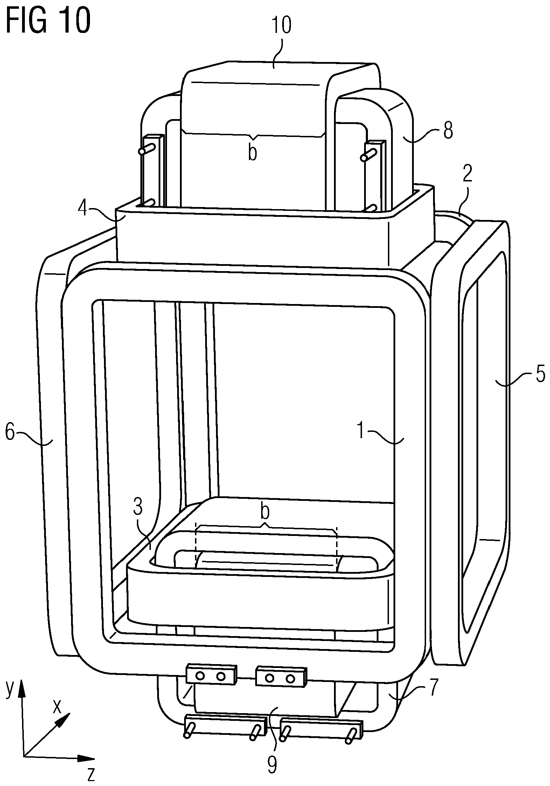

Fig. 10 zeigt in perspektivischer Ansicht ein konkretes Ausführungsbeispiel einer Spulenanordnung gemäß Fig.3, wobei die in Fig. 10 verwendeten Spulen in allen vorangegangenen Ausführungsformen gemäß Fig. 1 bis Fig. 9 einsetzbar sind. Man erkennt, dass in diesem Ausführungsbeispiel die einzelnen Spulen 1 bis 10 als Flächenspulen ausgebildet sind, wobei neben den Spulen 7 und 8 insbesondere die Spulen 9 und 10 als breite Flächenspulen ausgebildet sind, deren jeweilige Breite b deutlich größer ist als die Dicke bzw. Wickelhöhe der Spulen. Breite Flächenspulen werden vorzugsweise auch in solchen Ausführungsformen verwendet, bei denen ein Spulenpaar durch eine einzelne, zentral angeordnete Spule ersetzt wird. Zumindest diese zentral angeordnete Spule, welche in den vorangegangenen Ausführungsbeispielen mit dem Bezugszeichen 5' bezeichnet ist, wird dabei als breite Flächenspule ausgebildet.

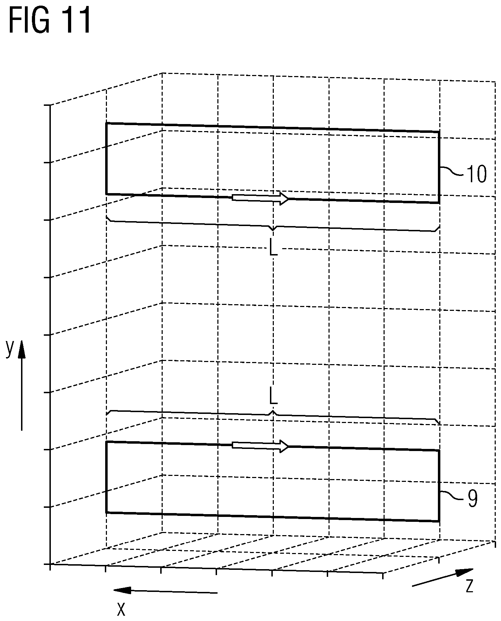

Fig. 11 zeigt einen entsprechenden Stromfluss durch die in Fig. 10 gezeigten Spulen 9 und 10. Der Feldgradient des entsprechenden Nebendiagonalelements der Gradientenmatrix wird dabei nur durch den Primärleiter bewirkt, der in Fig. 11 mit dem Bezugszeichen L verdeutlicht ist. Der Rest des Leiters in der Spule, d.h. der Rückleiter, sollte möglichst weit entfernt vom Arbeitsraum angeordnet sein. Ferner sollte der stromführende Teil der Spule möglichst nah am Arbeitsraum wie möglich angeordnet sein, woraus sich die bevorzugte Ausgestaltung der Spule 9 bzw. 10 als breite Flächenspulen ergibt.

Basierend auf den oben beschriebenen Varianten der Erfindung werden minimale Spulen-System-Konfigurationen geschaffen, welche sich entsprechend den Anforderungen an die Freiheitsgrade der Bewegung eines magnetischen Objekts, insbesondere einer mit einem Permanentmagneten ausgestatteten EndoskopieKapsel, mit geringem Aufwand, d.h. mit einer möglichst geringen Anzahl an Leistungsverstärkern bzw. Spulen, realisieren lassen. Insbesondere beruht die Erfindung dabei auf der Erkenntnis, dass sich bei entsprechenden Navigationsanforderungen die Anzahl der Leistungsverstärker zur Ansteuerung der Spulen bzw. die Anzahl der Spulen reduzieren lässt.

**Patentansprüche**

1.	Spulenanordnung zur berührungslosen Führung eines magnetischen Objektes, insbesondere einer Endoskopiekapsel, mit einem magnetischen Dipolmoment (m) in einem Arbeitsraum in einem kartesischen Koordinatensystem mit drei Achsen (x, y, z) umfassend eine horizontale erste Achse (x), eine horizontale zweite Achse (z) und eine vertikale dritte Achse (y), wobei die Spulenanordnung eine Mehrzahl von Einzelspulen (1, 2, ..., 12) zur Erzeugung von Komponenten ($B_x$, $B_y$, $B_z$) eines Magnetfelds (B) und von magnetischen Gradientenfeldern und mehrere Ansteuereinheiten zur Steuerung der Bestromung der Einzelspulen (1, 2, ..., 12) umfasst, wobei:

- die Mehrzahl von Einzelspulen (1, 2, ..., 12) eine Mehrzahl von ersten Einzelspulen (1, 2, ...,6) umfasst, wobei der Arbeitsraum gesehen aus der Richtung der Längsachse einer jeweiligen ersten Einzelspule (1, 2, ..., 6) innerhalb des Umfangs der jeweiligen ersten Einzelspule (1, 2, ..., 6) liegt;
- einer oder mehreren der Achsen (x, y, z) jeweils zumindest ein erstes Spulenpaar aus zwei ersten Einzelspulen (1, 2, ..., 6) zugeordnet ist, welche entlang der jeweiligen Achse (x, y, z) an gegenüberliegenden Seiten des Arbeitsraums in im Wesentlichen parallelen Ebenen angeordnet sind;
- einer oder mehreren der Achsen (x, y, z) nur jeweils eine einzelne erste Einzelspule (5') oder keine erste Einzelspule (5') zugeordnet ist, wobei der Arbeitsraum zumindest teilweise von dem Umfang einer jeweiligen ersten Einzelspule (1, 2, ..., 6) umgeben ist;
- die Mehrzahl von Einzelspulen eine Mehrzahl von zweiten Einzelspulen (7, 8, ..., 12) umfasst, wobei der Arbeitsraum gesehen aus der Richtung der Längsachse einer jeweiligen zweiten Einzelspule (7, 8, ..., 12) außerhalb des Umfangs der jeweiligen zweiten Einzelspule liegt;
- einer oder mehreren der Achsen (x, y, z) jeweils zumindest ein zweites Spulenpaar aus zwei zweiten Einzelspulen (7, 8, ..., 12) zugeordnet ist, welche im Wesentlichen in einer gemeinsamen Ebene entlang der jeweiligen Achse (x, y, z) an gegenüberliegenden Seiten des Arbeitsraums angeordnet sind.

2. Spulenanordnung nach Anspruch 1, wobei zumindest zwei Einzelspulen (1, 2, ..., 12) durch eine gemeinsame Ansteuereinheit ansteuerbar sind.

3. Spulenanordnung nach Anspruch 1 oder 2, welche genau elf Einzelspulen (1, 2, ..., 12) umfasst, wobei vorgesehen sind:

   - zwei erste Spulenpaare (1, 2, 3, 4), wobei eines der ersten Spulenpaare (1, 2) der ersten Achse (x) zugeordnet ist und das andere der ersten Spulenpaare (3, 4) der dritten Achse (y) zugeordnet ist;
   - eine einzelne erste Einzelspule (5'), welche der zweiten Achse (z) zugeordnet ist;
   - ein zweites Spulenpaar (11, 12), welches der ersten Achse (x) zugeordnet ist und in einer Ebene angeordnet ist, welche durch die erste und dritte Achse (x, y) aufgespannt ist;
   - zwei zweite Spulenpaare (7, 8, 9, 10), welche der dritten Achse (y) zugeordnet sind und welche in Ebenen im Wesentlichen senkrecht zueinander angeordnet sind, wobei eine der Ebenen durch die erste und dritte Achse (x, y) aufgespannt ist und die andere der Ebenen durch die zweite und dritte Achse (z, y) aufgespannt ist.

4. Spulenanordnung nach Anspruch 1, welche genau elf Einzelspulen (1, 2, ..., 12) umfasst, wobei vorgesehen sind:

   - zwei erste Spulenpaare (1, 2, 3, 4), wobei eines der ersten Spulenpaare (1, 2) der ersten Achse (x) zugeordnet ist und das andere der ersten Spulenpaare (3, 4) der dritten Achse (y) zugeordnet ist;
   - eine einzelne erste Einzelspule (5'), welche der zweiten Achse (z) zugeordnet ist;
   - ein zweites Spulenpaar (11, 12), welches der ersten Achse (x) zugeordnet ist und in einer Ebene angeordnet ist, welche durch die erste und dritte Achse (x, y) aufgespannt ist;
   - ein zweites Spulenpaar (7, 8), welches der ersten Achse (x) zugeordnet ist und in einer Ebene angeordnet ist, welche durch die erste und zweite Achse (x, z) aufgespannt ist;
   - ein zweites Spulenpaar (9, 10), welches der dritten Achse (y) zugeordnet ist und in einer Ebene angeordnet ist, welche durch die erste und dritte Achse (x, y) aufgespannt ist.

5. Spulenanordnung nach Anspruch 3 oder 4, welche genau acht Ansteuereinheiten umfasst, wobei:

   - alle ersten Einzelspulen (1, 2, ..., 6) durch separate Ansteuereinheiten ansteuerbar sind;
   - die zweiten Spulenpaare (7, 8, ..., 12) jeweils durch eine gemeinsame Ansteuereinheit ansteuerbar sind.

6. Spulenanordnung nach Anspruch 1 oder 2, welche genau neun Einzelspulen (1, 2, ..., 12) umfasst, wobei vorgesehen sind:

   - zwei erste Spulenpaare (1, 2, 3, 4), wobei eines der ersten Spulenpaare (1, 2) der ersten Achse (x) zugeordnet ist und das andere der ersten Spulenpaare (3, 4) der dritten Achse (y) zugeordnet ist;
   - eine einzelne erste Einzelspule (5'), welche der zweiten Achse (z) zugeordnet ist;
   - zwei zweite Spulenpaare (7, 8, 9, 10), welche der dritten Achse (y) zugeordnet sind und welche in Ebenen im Wesentlichen senkrecht zueinander angeordnet sind, wobei eine der Ebenen durch die erste und dritte Achse (x, y) aufgespannt ist und die andere der Ebenen durch die zweite und dritte Achse (z, y) aufgespannt ist.

7. Spulenanordnung nach Anspruch 1 oder 2, welche genau neun Einzelspulen (1, 2, ..., 12) umfasst, wobei vorgesehen sind:

   - zwei erste Spulenpaare (1, 2, 3, 4), wobei eines der ersten Spulenpaare (1, 2) der ersten Achse (x) zugeordnet ist und das andere der ersten Spulenpaare (3, 4) der dritten Achse (y) zugeordnet ist;
   - eine einzelne erste Einzelspule (5'), welche der zweiten Achse (z) zugeordnet ist;
   - ein zweites Spulenpaar (7, 8), welches der ersten Achse (x) zugeordnet ist und in einer Ebene angeordnet ist, welche durch die erste und zweite Achse (x, z) aufgespannt ist;
   - ein zweites Spulenpaar (9, 10), welches der dritten Achse (y) zugeordnet ist und in einer Ebene angeordnet ist, welche durch die erste und dritte Achse (x, y) aufgespannt ist.

8. Spulenanordnung nach Anspruch 6 oder 7, welche genau sieben Ansteuereinheiten umfasst, wobei:

- alle ersten Einzelspulen (1, 2, ..., 6) durch separate Ansteuereinheiten ansteuerbar sind;
- die zweiten Spulenpaare (7, 8, ..., 12) jeweils durch eine gemeinsame Ansteuereinheit ansteuerbar sind.

9. Spulenanordnung nach Anspruch 1 oder 2, welche genau acht Einzelspulen (1, 2, ..., 12) umfasst, wobei vorgesehen sind:

- ein erstes Spulenpaar (3, 4), welches der dritten Achse (y) zugeordnet ist;
- ein zweites Spulenpaar (11, 12), welches der ersten Achse (x) zugeordnet ist und in einer Ebene angeordnet ist, welche durch die erste und dritte Achse (x, y) aufgespannt ist;
- zwei zweite Spulenpaare (7, 8, 9, 10), welche der dritten Achse (y) zugeordnet sind und welche in Ebenen im Wesentlichen senkrecht zueinander angeordnet sind, wobei eine der Ebenen durch die erste und dritte Achse (x, y) aufgespannt ist und die andere der Ebenen durch die zweite und dritte (z, y) Achse aufgespannt ist.

10. Spulenanordnung nach Anspruch 1 oder 2, welche genau acht Einzelspulen (1, 2, ..., 12) umfasst, wobei vorgesehen sind:

- ein erstes Spulenpaar (1, 2), welches der ersten Achse (y) zugeordnet sind;
- ein zweites Spulenpaar (11, 12), welches der ersten Achse (x) zugeordnet ist und in einer Ebene angeordnet ist, welche durch die erste und dritte Achse (x, y) aufgespannt ist;
- ein zweites Spulenpaar (7, 8), welches der ersten Achse (x) zugeordnet ist und in einer Ebene angeordnet ist, welche durch die erste und zweite Achse (x, z) aufgespannt ist;
- ein zweites Spulenpaar (9, 10), welches der dritten Achse (y) zugeordnet ist und in einer Ebene angeordnet ist, welche durch die erste und dritte Achse (x, y) aufgespannt ist.

11. Spulenanordnung nach Anspruch 9 oder 10, welche genau sieben Ansteuereinheiten umfasst, wobei alle zweiten Einzelspulen (7, 8, ..., 12) durch separate Ansteuereinheiten ansteuerbar sind und die zwei ersten Einzelspulen (1, 2; 3, 4) des ersten Spulenpaars durch eine gemeinsame Ansteuereinheit ansteuerbar sind.

12. Spulenanordnung nach Anspruch 9 oder 10, welche genau sechs Ansteuereinheiten umfasst, wobei

- die zweiten Einzelspulen des zweiten Spulenpaars (11, 12), welches der ersten Achse (x) zugeordnet ist und in einer Ebene angeordnet ist, welche durch die erste und dritte Achse (x, y) aufgespannt ist, durch eine gemeinsame Ansteuereinheit ansteuerbar sind;
- die zwei ersten Einzelspulen (1, 2; 3, 4) des ersten Spulenpaars durch eine gemeinsame Ansteuereinheit ansteuerbar sind;
- die restlichen Einzelspulen (7, 8, 9, 10) durch separate Ansteuereinheiten ansteuerbar sind.

13. Spulenanordnung zur berührungslosen Führung eines magnetischen Objekts, insbesondere einer Endoskopiekapsel, mit einem magnetischen Dipolmoment (m) in einem Arbeitsraum in einem kartesischen Koordinatensystem mit drei Achsen (x, y, z) umfassend eine horizontale erste Achse (x), eine horizontale zweite Achse (z) und eine vertikale dritte Achse (y), wobei die Spulenanordnung eine Mehrzahl von Einzelspulen (1, 2, ..., 12) zur Erzeugung von Komponenten ($B_x$, $B_y$, $B_z$) eines Magnetfelds (B) und von magnetischen Gradientenfeldern und mehrere Ansteuereinheiten zur Steuerung der Bestromung der Einzelspulen (1, 2, ..., 12) umfasst, wobei:

- die Mehrzahl von Einzelspulen (1, 2, ..., 12) ausschließlich eine Mehrzahl von ersten Einzelspulen (1, 2, ..., 6) umfasst, wobei der Arbeitsraum gesehen aus der Richtung der Längsachse einer jeweiligen ersten Einzelspule (1, 2, ..., 6) innerhalb des Umfangs der jeweiligen ersten Einzelspule (1, 2, ..., 6) liegt;
- einer oder mehreren der Achsen (x, y, z) jeweils zumindest ein erstes Spulenpaar aus zwei ersten Einzelspulen (1, 2, ..., 6) zugeordnet ist, welche entlang der jeweiligen Achse (x, y, z) an gegenüberliegenden Seiten des Arbeitsraums in im Wesentlichen parallelen Ebenen angeordnet sind;
- die ersten Einzelspulen (1, 2, ..., 6) zumindest eines ersten Spulenpaars (5, 6) durch eine gemeinsame Ansteuereinheit ansteuerbar sind;
- die Spulenanordnung genau sechs erste Einzelspulen (1, 2, ..., 6) umfasst, wobei jeder Achse (x, y, z) ein erstes Spulenpaar zugeordnet ist;
- die Spulenanordnung genau fünf Ansteuereinheiten umfasst, wobei die zwei ersten Einzelspulen (1, 2, 3, 4) eines ersten Spulenpaars durch eine gemeinsame Ansteuereinheit ansteuerbar sind die restlichen ersten Einzelspulen (5, 6) durch separate Ansteuereinheiten ansteuerbar sind.

**EP 2 408 390 B1**

14. Spulenanordnung zur berührungslosen Führung eines magnetischen Objekts, insbesondere einer Endoskopiekapsel, mit einem magnetischen Dipolmoment (m) in einem Arbeitsraum in einem kartesischen Koordinatensystem mit drei Achsen (x, y, z) umfassend eine horizontale erste Achse (x), eine horizontale zweite Achse (z) und eine vertikale dritte Achse (y), wobei die Spulenanordnung eine Mehrzahl von Einzelspulen (1, 2, ..., 12) zur Erzeugung von Komponenten ($B_x$, $B_y$, $B_z$) eines Magnetfelds (B) und von magnetischen Gradientenfeldern und eine Mehrzahl von Ansteuereinheiten zur Steuerung der Bestromung der Einzelspulen umfasst, wobei:

- die Mehrzahl von Einzelspulen (1, 2, ..., 12) ausschließlich eine Mehrzahl von ersten Einzelspulen (1, 2, ..., 6) umfasst, wobei der Arbeitsraum gesehen aus der Richtung der Längsachse einer jeweiligen ersten Einzelspule (1, 2, ..., 6) innerhalb des Umfangs der jeweiligen ersten Einzelspule (1, 2, ..., 6) liegt;
- einer oder mehreren der Achsen (x, y, z) jeweils zumindest ein erstes Spulenpaar aus zwei ersten Einzelspulen (1, 2, ..., 6) zugeordnet ist, welche entlang der jeweiligen Achse (x, y, z) an gegenüberliegenden Seiten des Arbeitsraums in im Wesentlichen parallelen Ebenen angeordnet sind;
- einer oder mehreren der Achsen (x, y, z) nur jeweils eine einzelne erste Einzelspule (5') zugeordnet ist, wobei der Arbeitsraum zumindest teilweise durch den Umfang einer jeweiligen einzelnen ersten Einzelspule (1, 2, ..., 6) umgeben ist.

15. Spulenanordnung nach Anspruch 14, wobei alle ersten Einzelspulen (1, 2, ..., 6) durch separate Ansteuereinheiten ansteuerbar sind.

16. Spulenanordnung nach Anspruch 14 oder 15, welche genau fünf erste Einzelspulen (1, 2, 3, 4, 5') umfasst.

17. Spulenanordnung nach einem der vorhergehenden Ansprüche, wobei die Ansteuereinheiten Leistungsverstärker umfassen.

18. Spulenanordnung nach einem der vorhergehenden Ansprüche, wobei die Einzelspulen (1, 2, ..., 12) zumindest teilweise Ringspulen und/oder Sattelspulen umfassen.

19. Spulenanordnung nach einem der vorhergehenden Ansprüche, wobei die Einzelspulen (1, 2, ..., 12) zumindest teilweise Flächenspulen umfassen, insbesondere mit einer Breite in Längsrichtung, welche größer als Dicke der Spule ist, wobei die Breite vorzugsweise mindestens das Fünffache der Dicke beträgt.

20. Spulenanordnung nach einem der vorhergehenden Ansprüche, mit einem Patiententisch, auf dem im Betrieb der Spulenanordnung der zu untersuchende Patient liegt, wobei der Patiententisch derart angeordnet ist, dass sich die Längsachse eines Patienten auf dem Patiententisch im Wesentlichen in Richtung der zweiten Achse (z) erstreckt.

**Claims**

1. Coil assembly for contactless guidance of a magnetic object, in particular an endoscopy capsule, with a magnetic dipole moment (m) in a workspace in a Cartesian coordinate system with three axes (x, y, z) comprising a horizontal first axis (x), a horizontal second axis (z) and a vertical third axis (y), wherein the coil assembly has a plurality of individual coils (1, 2, ..., 12) for generating components ($B_x$, $B_y$, $B_z$) of a magnetic field (B) and magnetic gradient fields, and multiple control units for controlling the energisation of the individual coils (1, 2, ..., 12), wherein:

- the plurality of individual coils (1, 2, ..., 12) comprises a plurality of first individual coils (1, 2, ..., 6), wherein the workspace, when viewed from the direction of the longitudinal axis of a respective first individual coil (1, 2, ..., 6), lies within the circumference of the respective first individual coil (1, 2, ..., 6);
- one or more of the axes (x, y, z) is in each case assigned at least one first coil pair made up of two first individual coils (1, 2, ..., 6), which are arranged along the respective axis (x, y, z) on opposite sides of the workspace in substantially parallel planes;
- one or more of the axes (x, y, z) is in each case assigned just a single first individual coil (5') or no first individual coil (5'), wherein the workspace is surrounded at least partially by the circumference of a respective first individual coil (1 , 2, ..., 6);
- the plurality of individual coils comprises a plurality of second individual coils (7, 8, ..., 12), wherein the workspace, when viewed from the direction of the longitudinal axis of a respective second individual coil (7, 8, ..., 12), lies outside the circumference of the respective second individual coil;
- one or more of the axes (x, y, z) is in each case assigned at least one second coil pair made up of two second individual coils (7, 8, ..., 12), which are substantially arranged in a common plane along the respective axis (x, y, z) on opposite sides of the workspace.

2. Coil assembly according to claim 1, wherein at least two individual coils (1, 2, ..., 12) can be controlled by a common control unit.

3. Coil assembly according to claim 1 or 2, which comprises exactly eleven individual coils (1, 2, ..., 12), wherein the following are provided:

- two first coil pairs (1, 2, 3, 4), wherein one of the first coil pairs (1, 2) is assigned to the first axis (x) and the other of the first coil pairs (3, 4) is assigned to the third axis (y);
- a single first individual coil (5') which is assigned to the second axis (z);
- a second coil pair (11, 12) which is assigned to the first axis (x) and is arranged in a plane which is spanned by the first and third axes (x, y);
- two second coil pairs (7, 8, 9, 10) which are assigned to the third axis (y) and which are arranged in planes substantially perpendicular to one another, wherein one of the planes is spanned by the first and third axes (x, y) and the other of the planes is spanned by the second and third axes (z, y).

4. Coil assembly according to claim 1, which comprises exactly eleven individual coils (1, 2, ..., 12), wherein the following are provided:

- two first coil pairs (1, 2, 3, 4), wherein one of the first coil pairs (1, 2) is assigned to the first axis (x) and the other of the first coil pairs (3, 4) is assigned to the third axis (y);
- a single first individual coil (5') which is assigned to the second axis (z);
- a second coil pair (11, 12) which is assigned to the first axis (x) and is arranged in a plane which is spanned by the first and third axes (x, y);
- a second coil pair (7, 8) which is assigned to the first axis (x) and is arranged in a plane which is spanned by the first and second axes (x, z);
- a second coil pair (9, 10) which is assigned to the third axis (y) and is arranged in a plane which is spanned by the first and third axes (x, y).

5. Coil assembly according to claim 3 or 4, which comprises exactly eight control units, wherein:

- all first individual coils (1, 2, ..., 6) can be controlled by separate control units;
- the second coil pairs (7, 8, ..., 12) can each be controlled by a common control unit.

6. Coil assembly according to claim 1 or 2, which comprises exactly nine individual coils (1, 2, ..., 12), wherein the following are provided:

- two first coil pairs (1, 2, 3, 4), wherein one of the first coil pairs (1, 2) is assigned to the first axis (x) and the other of the first coil pairs (3, 4) is assigned to the third axis (y);
- a single first individual coil (5') which is assigned to the second axis (z);
- two second coil pairs (7, 8, 9, 10) which are assigned to the third axis (y) and which are arranged in planes substantially perpendicular to one another, wherein one of the planes is spanned by the first and third axes (x, y) and the other of the planes is spanned by the second and third axes (z, y).

7. Coil assembly according to claim 1 or 2, which comprises exactly nine individual coils (1, 2, ..., 12), wherein the following are provided:

- two first coil pairs (1, 2, 3, 4), wherein one of the first coil pairs (1, 2) is assigned to the first axis (x) and the other of the first coil pairs (3, 4) is assigned to the third axis (y);
- a single first individual coil (5') which is assigned to the second axis (z);
- a second coil pair (7, 8) which is assigned to the first axis (x) and is arranged in a plane which is spanned by the first and second axes (x, z);
- a second coil pair (9, 10) which is assigned to the third axis (y) and is arranged in a plane which is spanned by the first and third axes (x, y).

8. Coil assembly according to claim 6 or 7, which comprises exactly seven control units, wherein:

- all first individual coils (1, 2, ..., 6) can be controlled by separate control units;
- the second coil pairs (7, 8, ..., 12) can each be controlled by a common control unit.

9. Coil assembly according to claim 1 or 2, which comprises exactly eight individual coils (1, 2, ..., 12), wherein the following are provided:

- a first coil pair (3, 4) which is assigned to the third axis (y);
- a second coil pair (11, 12) which is assigned to the first axis (x) and is arranged in a plane which is spanned by the first and third axes (x, y);
- two second coil pairs (7, 8, 9, 10) which are assigned to the third axis (y) and which are arranged in planes substantially perpendicular to one another, wherein one of the planes is spanned by the first and third axes (x, y) and the other of the planes is spanned by the second and third (z, y) axes.

10. Coil assembly according to claim 1 or 2, which comprises exactly eight individual coils (1, 2, ..., 12), wherein the following are provided:

- a first coil pair (1, 2) which is assigned to the first axis (y);
- a second coil pair (11, 12) which is assigned to the first axis (x) and is arranged in a plane which is spanned by the first and third axes (x, y);
- a second coil pair (7, 8) which is assigned to the first axis (x) and is arranged in a plane which is spanned by the first and second axes (x, z);
- a second coil pair (9, 10) which is assigned to the third axis (y) and is arranged in a plane which is spanned by the first and third axes (x, y).

11. Coil assembly according to claim 9 or 10, which comprises exactly seven control units, wherein all second individual coils (7, 8, ..., 12) can be controlled by separate control units and the two first individual coils (1, 2; 3, 4) of the first coil pair can be controlled by a common control unit.

12. Coil assembly according to claim 9 or 10, which comprises exactly six control units, wherein

- the second individual coils of the second coil pair (11, 12), which is assigned to the first axis (x) and is arranged in a plane which is spanned by the first and third axes (x, y), can be controlled by a common control unit;
- the two first individual coils (1, 2; 3, 4) of the first coil pair can be controlled by a common control unit;
- the remaining individual coils (7, 8, 9, 10) can be controlled by separate control units.

13. Coil assembly for contactless guidance of a magnetic object, in particular an endoscopy capsule, with a magnetic dipole moment (m) in a workspace in a Cartesian coordinate system with three axes (x, y, z) comprising a horizontal first axis (x), a horizontal second axis (z) and a vertical third axis (y), wherein the coil assembly has a plurality of individual coils (1, 2, ..., 12) for generating components ($B_x$, $B_y$, $B_z$) of a magnetic field (B) and magnetic gradient fields, and multiple control units for controlling the energisation of the individual coils (1, 2, ..., 12), wherein:

- the plurality of individual coils (1, 2, ..., 12) exclusively comprises a plurality of first individual coils (1, 2, ..., 6), wherein the workspace, when viewed from the direction of the longitudinal axis of a respective first individual coil (1, 2, ..., 6), lies within the circumference of the respective first individual coil (1, 2, ..., 6);
- one or more of the axes (x, y, z) is in each case assigned at least one first coil pair made up of two first individual coils (1, 2, ..., 6), which are arranged along the respective axis (x, y, z) on opposite sides of the workspace in substantially parallel planes;
- the first individual coils (1, 2, ..., 6) of at least one first coil pair (5, 6) can be controlled by a common control unit;
- the coil assembly comprises exactly six first individual coils (1, 2, ..., 6), wherein each axis (x, y, z) is assigned a first coil pair;
- the coil assembly comprises exactly five control units, wherein the two first individual coils (1, 2, 3, 4) of a first coil pair can be controlled by a common control unit and the remaining first individual coils (5, 6) can be controlled by separate control units.

14. Coil assembly for contactless guidance of a magnetic object, in particular an endoscopy capsule, with a magnetic dipole moment (m) in a workspace in a Cartesian coordinate system with three axes (x, y, z) comprising a horizontal first axis (x), a horizontal second axis (z) and a vertical third axis (y), wherein the coil assembly has a plurality of individual coils (1, 2, ..., 12) for generating components ($B_x$, $B_y$, $B_z$) of a magnetic field (B) and magnetic gradient fields, and a plurality of control units for controlling the energisation of the individual coils, wherein:

- the plurality of individual coils (1, 2, ..., 12) exclusively comprises a plurality of first individual coils (1, 2, ..., 6), wherein the workspace, when viewed from the direction of the longitudinal axis of a respective first individual coil (1, 2, ..., 6), lies within the circumference of the respective first individual coil (1, 2, ..., 6);
- one or more of the axes (x, y, z) is in each case assigned at least one first coil pair made up of two first individual coils (1, 2, ..., 6), which are arranged along the respective axis (x, y, z) on opposite sides of the workspace in substantially parallel planes;
- one or more of the axes (x, y, z) is in each case assigned just a single first individual coil (5'), wherein the workspace is surrounded at least partially by the circumference of a respective first individual coil (1, 2, ..., 6).

15. Coil assembly according to claim 14, wherein all first individual coils (1, 2, ..., 6) can be controlled by separate control units.

16. Coil assembly according to claim 14 or 15, which comprises exactly five first individual coils (1, 2, 3, 4, 5').

17. Coil assembly according to one of the preceding claims, wherein the control units comprise power amplifiers.

18. Coil assembly according to one of the preceding claims, wherein the individual coils (1, 2, ..., 12) at least partially comprise toroidal coils and/or saddle coils.

19. Coil assembly according to one of the preceding claims, wherein the individual coils (1, 2, ..., 12) at least partially comprise flat coils, in particular with a width in the longitudinal direction which is greater than the thickness of the coil, wherein the width is preferably at least five times the thickness.

20. Coil assembly according to one of the preceding claims, having a patient table on which the patient to be examined lies during operation of the coil assembly, wherein the patient table is arranged such that the longitudinal axis of a patient on the patient table extends substantially in the direction of the second axis (z).

## Revendications

1. Agencement de bobines pour le guidage sans contact d'un objet magnétique, notamment d'une capsule d'endoscopie, ayant un moment (M) dipolaire magnétique dans un espace de travail dans un système de coordonnées cartésien ayant trois axes (x, y, z), comprenant un premier axe (x) horizontal, un deuxième axe (z) horizontal et un troisième axe (y) vertical, l'agencement de bobines comprenant une pluralité de bobines (1, 2, 3..., 12) individuelles de production de composantes ($B_x$, $B_y$, $B_z$) d'un champ (B) magnétique et de champs magnétiques de gradient et plusieurs unités de commande pour commander l'alimentation en courant des bobines (1, 2, ..., 12) individuelles, dans lequel :

  - la pluralité de bobines 1, 2, ..., 12) individuelles comprend une pluralité de premières bobines (1, 2, ..., 6) individuelles, la chambre de travail, considérée dans la direction de l'axe longitudinal d'un première bobine (1, 2, ...,6) individuelle respective, étant à l'intérieur du pourtour de la première bobine (1, 2, ..., 6) individuelle respective.
  - à l'un ou à plusieurs des axes (x, y, z) est associé respectivement au moins une première paire de bobines composée de deux premières bobines (1, 2, ...,6) individuelles, qui sont disposées le long de l'axe (x, y, z) respectif, sur des côtés opposés de l'espace de travail dans des plans sensiblement parallèles ;
  - à l'un ou à plusieurs des axes (x, y, z) n'est associée que respectivement une première bobine (5') individuelle unique ou pas de première bobine (5') individuelle, l'espace de travail étant entouré au moins en partie du pourtour d'une première bobine (1, 2, ..., 6) individuelle respective ;
  - la pluralité de bobines individuelles comprend une pluralité de deuxièmes bobines (7, 8,..., 12) individuelles, l'espace de travail, considéré dans la direction de l'axe longitudinal d'une deuxième bobine (7, 8, ..., 12) individuelle respective, étant à l'extérieur du pourtour de la deuxième bobine individuelle respective.
  - à l'un ou à plusieurs des axes (x, y, z) est associé respectivement au moins une deuxième paires de bobines composée de deux deuxièmes bobines (7, 8, ...,12) individuelles, qui sont disposées dans un plan commun le long de l'axe (x, y, z) respectif sur des côtés opposés de l'espace de travail.

2. Agencement de bobines suivant la revendication 1, dans lequel au moins deux bobines (1, 2, ..., 12) individuelles peuvent être commandées par une unité de commande commune.

**3.** Agencement de bobines suivant la revendication 1 ou 2, qui comprend exactement onze bobines (1, 2, ..., 12) individuelles, dans lequel sont prévues :

- deux premières paires (1, 2, 3, 4) de bobines, l'une des premières paires (1, 2) de bobines étant associée au premier axe (x) et l'autre des première paires (3, 4) de bobine étant associée au troisième axe (y) ;
- une première bobine (5') individuelle unique, qui est associée au deuxième axe (z) ;
- une deuxième paire (11, 12) de bobines, qui est associée au premier axe (x) et qui est disposée dans un plan défini par le premier et le troisième axe (x, y) ;
- deux deuxièmes paires (7, 8, 9, 10) de bobines qui sont associées au troisième axe (y) et qui sont disposées dans des plans sensiblement perpendiculaires entre eux, l'un des plans étant défini par le premier et troisième axe (x, y) et l'autre plan étant défini par le deuxième et le troisième axe (z, y).

**4.** Agencement de bobines suivant la revendication 1, qui comprend exactement onze bobines (1, 2, ..., 12) individuelles, dans lequel sont prévues :

- deux premières paire (1, 2, 3, 4) de bobines, l'une des premières paires (1, 2) de bobines étant associée au premier axe (x) et l'autre des premières paires (3, 4) de bobines étant associée au troisième axe (y);
- une première bobine (5') individuelle unique, qui est associée au deuxième axe ;
- une deuxième paire (11, 12) de bobines, qui est associée au premier axe (x) et qui est disposée dans un plan défini par le premier et troisième axe (x, y) ;
- une deuxième paire (7, 8) de bobines, qui est associée au premier axe (x) et qui est disposée dans un plan défini par le premier et le deuxième axe (x, y) ;
- une deuxième paire (9, 10) de bobines, qui est associée au troisième axe (y) et qui est disposée dans un plan défini par le premier et troisième axes (x, y).

**5.** Agencement de bobines suivant la revendication 3 ou 4, qui comprend exactement huit unités de commande, dans lequel :

- toutes les premières bobines (1, 2, ..., 6) individuelles peuvent être commandées par des unités de commande distinctes ;
- les deuxièmes paires (7, 8, ..., 12) de bobines peuvent être excitées chacune par une unité d'excitation commune.

**6.** Agencement de bobines suivant la revendication 1 ou 2, qui comprend exactement neuf bobines (1, 2, ..., 12) individuelles, dans lequel sont prévues :

- deux premières paires (1, 23, 4) de bobines, l'une des premières paires (1, 2) de bobines étant associée au premier axe (x) et l'autre des premières paires (3, 4) de bobines étant associée au troisième axe (y) ;
- une première bobine (5') individuelle unique, qui est associée au deuxième axe (z) ;
- deux deuxièmes paires (7, 8, 9, 10) de bobines, qui sont associées au troisième axe (y) et qui sont disposées dans des plans sensiblement perpendiculaires entre eux, l'un des plans étant défini par le premier et le troisième axes (x, y) et l'autre plan étant défini par le deuxième et le troisième axes (z, y).

**7.** Agencement de bobines suivant la revendication 1 ou 2, qui comprend exactement neuf bobines (1, 2, ..., 12) individuelles dans lequel sont prévus :

- deux premières paires (1, 2, 3, 4) de bobines, l'une des premières paires (1, 2) de bobines étant associée au premier axe (x) et l'autre des premières paires (3, 4) de bobines étant associée au troisième axe (y) ;
- une première bobine (5') individuelle unique, qui est associée au deuxième axe (z) ;
- une deuxième paire (7, 8) de bobines, qui est associée au premier axe (x) et qui est disposée dans un plan défini par le premier et le deuxième axe (x, z) ;
- une deuxième paire (9, 10) de bobines, qui est associée au troisième axe (x) et qui est disposée dans un plan défini par le premier et le troisième axes (x, y).

**8.** Agencement de bobines suivant la revendication 6 ou 7, qui comprend exactement sept unités de commande, dans lequel :

- toutes les premières bobines (1, 2, ...,6) individuelles peuvent être commandées par des unités de commande distinctes ;
- les deuxièmes paires (7, 8, ..., 12) de bobine peuvent être commandées chacune par une unité de commande commune.

9. Agencement de bobines suivant la revendication 1 ou 2, qui comprend exactement huit bobines (1, 2, ..., 12) individuelles, dans lequel sont prévues :

- une première paire (3, 4) de bobines, qui est associée au troisième axe (y) ;
- une deuxième paire (11, 12) de bobines, qui est associée au premier axe (x) et qui est disposée dans un plan défini par le premier et troisième axes (x, y) ;
- deux deuxième paires (7, 8, 9, 10) de bobines, qui sont associées au troisième axe (y) et qui sont disposées dans des plans sensiblement perpendiculaires entre eux, l'un des plans étant défini par le premier et le troisième axes (x, y) et l'autre plan étant défini par le deuxième et troisième axes (z, y) .

10. Agencement de bobines suivant la revendication 1 ou 2, qui comprend exactement huit bobines (1, 2, ..., 12) individuelles dans lequel sont prévues :

- une première paire (1, 2) de bobines, qui est associée au premier axe (y) ;
- une deuxième paire (11, 12) de bobines, qui est associée au premier axe (x) et qui est disposée dans un plan défini par le premier et le troisième axes (x, y) ;
- une deuxième paire (7, 8) de bobines, qui est associée au premier axe (x) et qui est disposée dans un plan défini par le premier et le deuxième axes (x, z) ;
- une deuxième paire (9, 10) de bobines, qui est associée au troisième axe (y) et qui est disposée dans un plan défini par le premier et le troisième axes (x, y).

11. Agencement de bobines suivant la revendication 9 ou 10, qui comprend exactement sept unités de commande, dans lequel toutes les deuxièmes bobines (7, 8, ..., 12) individuelles peuvent être commandées par des unités de commandes distinctes et les deux premières bobines (1, 2, 3, 4) individuelles de la première paire de bobines peuvent être commandées par une unité de commande commune.

12. Agencement de bobines suivant la revendication 9 ou 10, qui comprend exactement six unités de commande, dans lequel

- les deuxièmes bobines individuelles de la deuxième paire (11, 12) de bobines, qui est associée au premier axe (x) et qui est disposée dans un plan défini par le premier et le troisième axes (x, y), peuvent être commandées par une unité de commande commune ;
- les deux premières bobines (1, 2, 3, 4) individuelles de la première paire de bobines peuvent être commandées par une unité de commande commune ;
- les bobines (7, 8, 9, 10) individuelles restantes peuvent être commandées par des unités de commande distinctes.

13. Agencement de bobines pour le guidage sans contact d'un objet magnétique, notamment d'une capsule d'endoscopie, ayant un moment (M) dipolaire magnétique dans un espace de travail dans un système de coordonnées cartésien ayant trois axes (x, y, z), comprenant un premier axe (x) horizontal, un deuxième axe (z) horizontal et un troisième axe (y) vertical, l'agencement de bobines comprenant une pluralité de bobines (1, 2, 3..., 12) individuelles de production de composantes ($B_X$, $B_Y$, $B_Z$) d'un champ (B) magnétique et de champs magnétiques de gradient et plusieurs unités de commande pour commander l'alimentation en courant des bobines (1, 2, ..., 12) individuelles, dans lequel :

- la pluralité de bobines (1, 2, ...,12) individuelles comprend exclusivement une pluralité de premières bobines (1, 2, ..., 6) individuelles, l'espace de travail, considéré dans la direction de l'axe longitudinal d'une première bobine (1, 2, ..., 6) individuelle respective, se trouvant à l'intérieur du pourtour de la première bobine (1, 2, ..., 6) individuelle respective ;
- à un ou à plusieurs des axes (x, y, z) est associée respectivement au moins une première paire de bobines composée de deux premières bobines (1, 2, ..., 6) individuelles, qui sont disposées le long de l'axe (x, y, z) respectif sur des côtés opposés de l'espace de travail dans des plans sensiblement parallèles ;
- les premières bobines (1, 2, ..., 6) individuelles d'au moins une première paire (5, 6) de bobines peuvent être commandées par une unité de commande commune ;
- l'agencement de bobines comprend exactement six premières bobines (1, 2, ..., 6) individuelles, une première paire de bobines étant associée à chaque axe (x, y, z) ;
- l'agencement de bobines comprend exactement cinq unités d'excitation, les deux premières bobines (1, 2, 3, 4) individuelles d'une première paire de bobines pouvant être commandées par une unité de commande commune, les premières bobines (5, 6) individuelles restantes pouvant être commandées par des unités de commande distinctes.

14. Agencement de bobines pour le guidage sans contact d'un objet magnétique, notamment d'une capsule d'endoscopie, ayant un moment (M) dipolaire magnétique dans un espace de travail dans un système de coordonnées cartésien ayant trois axes (x, y, z), comprenant un premier axe (x) horizontal, un deuxième axe (z) horizontal et un troisième axe (y) vertical, l'agencement de bobines comprenant une pluralité de bobines (1, 2, 3..., 12) individuelles de production de composantes ($B_X$, $B_Y$, $B_Z$) d'un champ (B) magnétique et de champs magnétiques de gradient et plusieurs unités de commande pour commander l'alimentation en courant des bobines (1, 2, ..., 12) individuelles, dans lequel :

   - la pluralité de bobines (1, 2, ..., 12) individuelles comprend exclusivement une pluralité de premières bobines (1, 2, ..., 6) individuelles, l'espace de travail, considéré dans la direction de l'axe longitudinal d'une première bobine (1, 2, ..., 6) individuelle respective, se trouvant à l'intérieur du pourtour de la première bobine (1, 2, ..., 6) individuelle respective ;
   - à l'un ou à plusieurs des axes (x, y, z) est associée respectivement au moins une première paire de bobines composée de deux premières bobines (1, 2, ..., 6) individuelles, qui sont disposées le long de l'axe (x, y, z) respectif sur des côtés opposés de l'espace de travail dans des plans sensiblement parallèles ;
   - à l'un ou à plusieurs des axes (x, y, z) n'est associée que respectivement une première bobine (5') individuelle unique, l'espace de travail étant entouré au moins en partie par le pourtour d'une première bobine (1, 2, ..., 6) individuelle unique respective.

15. Agencement de bobines suivant la revendication 14, dans lequel toutes les premières bobines (1, 2,..., 6) individuelles peuvent êtres commandées par des unités de commande distinctes.

16. Agencement de bobines suivant la revendication 14 ou 15, qui comprend exactement cinq premières bobines (1, 2, 3, 4, 5') individuelles.

17. Agencement de bobines suivant l'une des revendications précédentes, dans lequel les unités de commande comprennent des amplificateurs de puissance.

18. Agencement de bobines suivant l'une des revendications précédentes, dans lequel les bobines (1, 2,..., 12) individuelles comprennent au moins en partie des bobines toroïdales et/ou des bobines en sellette.

19. Agencement de bobines suivant l'une des revendications précédentes, dans lequel les bobines (1, 2, ..., 12) individuelles comprennent au moins des bobines plates, ayant notamment une largeur dans la direction longitudinale, qui est plus grande que l'épaisseur de la bobine, la largeur représentant de préférence au moins cinq fois à l'épaisseur.

20. Agencement de bobines suivant l'une des revendications précédentes, comprenant une table de patient, sur lequel, lorsque l'agencement de bobine est en fonctionnement, est allongé le patient à examiner, la table du patient étant disposée de manière à ce que l'axe longitudinal d'un patient sur la table de patient s'étende sensiblement dans la direction du deuxième axe (z).

FIG 1

FIG 2

FIG 3

FIG 4

## FIG 5

## FIG 6

FIG 7

FIG 8

FIG 9

# FIG 10

# FIG 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10340925 B3
- WO 2006092421 A1
- WO 2008004497 A1
- WO 2007110278 A1
- WO 2007077922 A1
- DE 102008004871235
- US 20030020810 A1
- US 20030181788 A1